# EUROPEAN PATENT APPLICATION

(11) **EP 4 368 198 A1**
(43) Date of publication of application: **15.05.2024**
(21) Application number: 22207323.1
(22) Date of filing: 14.11.2022
(51) Int. Cl.: A61K 39/00, A61K 39/104, A61K 39/12, C07K 19/00

(54) **COMPOSITION FOR DELIVERING A PROTEIN OR A POLYPEPTIDE TO A EUKARYOTIC CELL**

(71) Applicant: Klinikum der Universität München, 80337 München (DE)
(72) Inventor: TANG, Jie, 80337 München (DE); ROSENECKER, Joseph, 82541 Münsing (DE)
(74) Representative: Dr. Gassner & Partner mbB

(57) **Abstract**

The invention relates to a composition for delivering a protein or a polypeptide to a eukaryotic cell which composition comprises or consists of a ternary complex consisting of the following components: a synthetic compound, either an amphiphilic block copolymer or an amphiphilic lipopeptide, and either the protein or the polypeptide.

## Description

The present invention concerns a composition for delivering a protein or a polypeptide to a eukaryotic cell, the composition for use as a medicament, a use of the composition and a method for an *in vitro* delivery of a protein or a polypeptide to a eukaryotic cell in cell culture.

WO 2017/167870 A1 discloses a synthetic compound for enabling transfection of eukaryotic cells by means of an amphiphilic block copolymer. The synthetic compound comprises a peptide residue having at least one targeting sequence and a nucleic acid binding sequence. The nucleic acid binding sequence comprises or consists of at least four consecutive amino acid residues which are positively charged at pH 7.4. The targeting sequence is any sequence that causes a direction of the sequence to a position or a structure inside or on the surface of eukaryotic cells. The synthetic compound further comprises a hydrophobic moiety covalently linked to the peptide residue. The hydrophobic moiety comprises or consists of lipoic acid residue, lipoamide residue, a tetradecyl residue, a cholesteryl residue, or a further peptide residue having a sequence with more than 40 % amino acid residues with hydrophobic side chains.

In WO 2013/128423 A1, the use of at least one glycosylated tetrafunctional non-ionic amphiphilic block copolymer as immune adjuvant is disclosed. Furthermore, a vaccine composition comprising at least one antigen and, as immune adjuvant, at least one glycosylated tetrafunctional non-ionic amphiphilic block copolymer is disclosed.

The problem to be solved by the present invention is to provide a composition for delivering a protein or a polypeptide to a eukaryotic cell and for use as a medicament, a use of such a composition and a method for an *in vitro* delivery of a protein or a polypeptide to a eukaryotic cell in cell culture.

The problem of the present invention is solved by the subject-matter of claims 1, 12, 13 and 14. Embodiments of the invention are subject-matter of claims 2 to 11.

According to the invention a composition for delivering a protein or a polypeptide to a eukaryotic cell is provided. The composition comprises or consists of a ternary complex consisting of the following components: a synthetic compound, either an amphiphilic block copolymer or an amphiphilic lipopeptide, and either the protein or the polypeptide. Instead of the amphiphilic block copolymer or the amphiphilic lipopeptide, a polysorbate, a polyethylene glycol ether, polyvinyl pyrrolidone, 1,4-sorbitan, D-sorbitol or isosorbide can be comprised by the ternary complex.

Here and in the following chemical constituents of the composition mentioned in singular forms are to be understood as a plurality of molecules of these constituents. For example, the term "providing the synthetic compound" means that a plurality of molecules of the synthetic compound are provided and the term "mixing the synthetic compound with the amphiphilic block copolymer or the amphiphilic lipopeptide" means that a plurality of molecules of the synthetic compound are mixed with a plurality of molecules of the amphiphilic block copolymer or a plurality of molecules of the amphiphilic lipopeptide". Consequently, the resulting composition comprises a plurality of the complexes.

The synthetic compound comprises or consists of a peptide residue and a hydrophobic moiety covalently linked to the peptide residue. The peptide residue is protein binding or polypeptide binding by comprising or consisting of a protein binding or polypeptide binding moiety. The hydrophobic moiety comprises or consists of a lipoic acid residue, a lipoamide residue, a tetradecyl residue, a cholesteryl residue, or a further peptide residue being hydrophobic by having a sequence consisting of any of SEQ ID NO. 21 to 29 or consisting of a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID NO. 21 to 29. The sequence similarity is calculated according to the EMBOSS needle algorithm having an open gap penalty of 10.0, an extended gap penalty of 0.5 and using the Blosum 62 matrix.

The inventors found that the common technical feature of all of the sequences of the peptide residue mentioned is that they are able to form the ternary complex of the composition according to the invention in combination with the hydrophobic moiety. The common technical feature of all of the sequences of the further peptide residue is that they provide such a hydrophobicity that the formation of the ternary complex is enabled. The ternary complex is able to deliver the protein or the polypeptide to a eukaryotic cell.

Sequence similarity in the context of the present invention is determined according to the EMBOSS needle algorithm. The EMBOSS needle algorithm is the standard algorithm for aligning a first amino acid sequence, in particular a first protein, a first polypeptide residue or a first oligopeptide residue, and a second amino acid sequence, in particular a second protein, a second polypeptide residue or a second oligopeptide residue, over the whole length of both the first amino acid sequence and the second amino acid sequence. The EMBOSS needle algorithm implements the Needleman-Wunsch algorithm (Needleman S.B. and Wunsch C.D., 1970 J. Mol. Biol. 48, 443 - 453), wherein a penalty for a gap of n positions is computed according to the following formula: open gap penalty + n - 1 x extended gap penalty.

The entire length of the first amino acid sequence and the second amino acid sequence is aligned, and there is no penalty for hanging ends of the overlap.

A "gap" designates one or more amino acid residue(s) not being identical in the aligned sequences. The open gap penalty in the context of the present invention is 10.0. The extended gap penalty in the context of the present invention is 0.5. The scoring matrix for comparing amino acid similarities in the context of the present invention is the Blosum 62 matrix. The open gap penalty, the extended gap penalty and the Blosum 62 matrix are standard parameters used in the art. In pairwise sequence alignment, the open gap penalty refers to the penalty for opening a gap in the alignment. The open gap penalty does not penalize terminal gaps. In pairwise sequence alignment, the extended gap penalty refers to the penalty for extending a gap by one residue. The extended gap penalty does not penalize terminal gaps. Sequence alignments can be performed with these parameters, e.g. via publicly available free tools. For example, the EBI (https://www.ebi.ac.uk/Tools/psa/emboss_needle/) offers a free pairwise sequence alignment service with the parameters of the present invention via its assortment of internet tools.

The protein binding or polypeptide binding moiety may be a cationic moiety that binds non-specifically to an anionic moiety of the protein or the polypeptide, wherein the protein binding or polypeptide binding moiety may have a sequence consisting of any of SEQ ID NO. 1 to 20 or a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID NO. 1 to 20, wherein the sequence similarity is calculated according to the EMBOSS needle algorithm having an open gap penalty of 10.0, an extended gap penalty of 0.5 and using the Blosum 62 matrix.

The protein binding or polypeptide binding moiety may be an anionic moiety that binds non-specifically to a cationic moiety of the protein or the polypeptide, wherein the protein binding or polypeptide binding moiety may have a sequence consisting of any of SEQ ID NO. 104 to 136 or a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID NO. 104 to 136, wherein the sequence similarity is calculated according to the EMBOSS needle algorithm having an open gap penalty of 10.0, an extended gap penalty of 0.5 and using the Blosum 62 matrix.

The protein binding or polypeptide binding moiety may be a moiety that binds specifically to a moiety of the protein or the polypeptide, wherein the protein binding or polypeptide binding moiety may comprise or consist of at least one and at most four His tag(s), wherein the His tag has a sequence according to SEQ ID NO. 137 and the protein or the polypeptide comprises an antibody or an antigen binding fragment thereof specifically binding to the His tag(s), or wherein the protein binding or polypeptide binding moiety may comprise or consist of at least one and at most four FLAG tag(s), wherein the FLAG tag has a sequence according to SEQ ID NO. 138 and the protein or the polypeptide comprises an antibody or an antigen binding fragment thereof specifically binding to the FLAG tag(s), or wherein the protein binding or polypeptide binding moiety may comprise or consist of at least one and at most four HA tag(s), wherein the HA tag has a sequence according to SEQ ID NO. 139 and the protein or the polypeptide comprises an antibody or an antigen binding fragment thereof specifically binding to the HA tag(s), or wherein the protein binding or polypeptide binding moiety may comprise or consist of at least one and at most four myc tag(s), wherein the myc tag has a sequence according to SEQ ID NO. 140 and the protein or the polypeptide comprises an antibody or an antigen binding fragment thereof specifically binding to the myc tag(s), or wherein the protein binding or polypeptide binding moiety may comprise or consist of at least one and at most four V5 tag(s); wherein the V5 tag has a sequence according to SEQ ID NO. 141 and the protein or the polypeptide comprises an antibody or an antigen binding fragment thereof specifically binding to the V5 tag(s), or wherein the protein binding or polypeptide binding moiety may comprise or consist of at least one and at most four Avi tag(s); wherein the Avi tag has a sequence according to SEQ ID NO. 142 and the protein or the polypeptide comprises an antibody or an antigen binding fragment thereof specifically binding to the Avi tag(s), or wherein the protein binding or polypeptide binding moiety may comprise or consist of at least one and at most four Strep-tag(s) II; wherein the Strep-tag II has a sequence according to SEQ ID NO. 143 and the protein or the polypeptide comprises biotin, an antibody or an antigen binding fragment thereof specifically binding to the Strep-tag(s) II, or wherein the protein binding or polypeptide binding moiety may comprise or consist of at least one and at most four S tag(s); wherein the S tag has a sequence according to SEQ ID NO. 144 and the protein or the polypeptide comprises an antibody or an antigen binding fragment thereof specifically binding to the S tag(s), or wherein the protein binding or polypeptide binding moiety may comprise or consist of at least one and at most four E tag(s), wherein the E tag has a sequence according to SEQ ID NO. 145 and the protein or the polypeptide comprises an antibody or an antigen binding fragment thereof specifically binding to the E tag(s).

The peptide residue may further comprise at least one targeting moiety. The targeting moiety is any moiety that causes a direction of the synthetic compound to a position or a structure inside or on the surface of eukaryotic cells. However, even if the peptide residue does not comprise a targeting moiety, the ternary complex is taken up by any eukaryotic cell. The targeting moiety may be a ligand for a specific receptor on the surface of specific eukaryotic cells, such as a Fc receptor on the surface of an immune cell, or a sequence which specifically binds to a structure on the surface of or inside specific eukaryotic cells. In particular the targeting moiety comprises or consists of a nuclear localization signal (NLS) sequence, a skeletal muscle cell ligand, a myocardium ligand, an epithelium cell ligand, an immune cell ligand, an endothelial cell ligand or a skin cell ligand. The targeting moiety may in particular comprise or consist of an immune cell ligand. The epithelium cell ligand may be a respiratory epithelium cell ligand or a microfold cell ligand. The immune cell ligand may be a macrophage cell ligand, a dendritic cell ligand, a T cell ligand or a B cell ligand. The immune cell ligand may be in particular a dendritic cell ligand. The endothelial cell ligand may be a lung endothelial cell ligand or a liver endothelial cell ligand. The skin cell ligand may be a dermal fibroblast cell ligand or a keratinocyte cell ligand.

The targeting moiety/moieties allow(s) it to specifically direct the protein or the polypeptide to the cells to which the protein or the polypeptide should be delivered and/or to a specific compartment inside of cells in which compartment the protein or the polypeptide shall exert its effect. The synthetic compound in the composition according to the invention may comprise two or more targeting sequences, e. g. an epithelium ligand sequence for directing the protein or the polypeptide to epithelium cells and a dendritic cell ligand directing the protein or the polypeptide to dendritic cells, or an epithelium ligand sequence for directing the protein or the polypeptide to epithelium cells and a NLS for directing the protein or the polypeptide to the nucleus of these epithelium cells. In particular, the synthetic compound may comprise exactly one, exactly two, exactly three or exactly four of the above mentioned specific targeting moieties.

The targeting moiety may comprise or consist of mannose or of an oligopeptide or polypeptide residue having a sequence consisting of any of SEQ ID NO. 30 to 51 or a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID No. 30 to 51. The sequence similarity is calculated according to the EMBOSS needle algorithm having an open gap penalty of 10.0, an extended gap penalty of 0.5 and using the Blosum 62 matrix.

The synthetic compound as specified according to the invention may be modified for preventing a degradation inside an organism, e. g. by PEGylation, i. e. a covalent or non-covalent attachment of polyethylene glycol to the synthetic compound. The modification for preventing a degradation inside an organism may be covalently linked to any part of the synthetic compound, in particular to the amino terminus of the synthetic compound or to the carboxy terminus of the synthetic compound or to any side chain of any amino acid residue of the synthetic compound, in particular the side chain of lysine, glutamic acid or aspartic acid.

Even though poloxamine-based block copolymers are promising non-viral gene delivery vectors for *in vivo* application, the inventors of the present invention recognized that they are rather poor protein delivery agents in delivering a protein or a polypeptide into cells, in particular cultured cells.

The inventors found that the synthetic compound, the amphiphilic block copolymer or the amphiphilic lipopeptide, and the protein or the polypeptide form a ternary complex which ternary complex very efficiently delivers the protein or the polypeptide to the eukaryotic cell. Furthermore, the inventors found that the synthetic compound and the protein or the polypeptide form a binary complex which binary complex also efficiently delivers the protein or the polypeptide to the eukaryotic cell. However, lung tissue toxicity after intratracheal administration of the binary complex is relatively high. A relatively low lung tissue toxicity was observed after intratracheal administration of the ternary complex. Furthermore, the inventors of the present invention found that lung tissue toxicity is even lower, when the synthetic compound as specified according to the invention comprises at least one targeting moiety, in particular an epithelial cell ligand, in particular a lung epithelial cell ligand, or an immune cell ligand, in particular a dendritic cell ligand.

The effect of the protein binding or polypeptide binding moiety is that it causes a condensation of proteins or polypeptides and facilitates an efficient cytoplasmic localization. The protein binding or polypeptide binding moiety may be a moiety that binds non-specifically to the protein or the polypeptide or a moiety of the protein or the polypeptide or may be a moiety that binds specifically to the protein or the polypeptide or a moiety of the protein or the polypeptide. If the protein binding or polypeptide binding moiety is a moiety that binds non-specifically to the protein or the polypeptide or a moiety of the protein or the polypeptide, the protein binding or polypeptide binding moiety may either be a cationic moiety that binds non-specifically to an anionic moiety of the protein or the polypeptide or an anionic moiety that binds non-specifically to a cationic moiety of the protein or the polypeptide.

Furthermore, the inventors of the present invention found that the protein binding or polypeptide binding moiety may improve an immune response directed against the protein or the polypeptide, in particular if the protein or the polypeptide is delivered to an immune cell, in particular to a dendritic cell, a macrophage, an airway epithelial cell, a T cell or a B cell.

The protein binding or polypeptide binding moiety may comprise at least two, in particular at least three, in particular at least four, oligopeptide or polypeptide residues having a sequence consisting of any of SEQ ID NO. 1 to 20 and 104 to 145 or of at least two, in particular at least three, in particular at least four, oligopeptide or polypeptide residues having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID No. 1 to 20 and 104 to 145, wherein any two of these oligopeptide or polypeptide residues are consecutively linked directly or by at most two linking amino acid residues selected from a group consisting of lysine, arginine, histidine, glycine, alanine, valine, cysteine, proline, leucine, isoleucine, methionine, tryptophan, phenylalanine residue and their derivatives. Alternatively, the protein binding or polypeptide binding moiety may consist of two, three, four, five or six of said oligopeptide or polypeptide residues having said sequence, optionally together with said linking amino acid residues. Consecutively linked sequences are to be understood as sequences that form one single new amino acid sequence after formation of the link. The sequence similarity is calculated according to the EMBOSS needle algorithm having an open gap of 10.0, an extended gap penalty of 0.5 and using the Blosum 62 matrix.

The protein binding or polypeptide binding moiety may consist of 4 to 33, in particular of 5 to 32, in particular of 6 to 31, in particular of 7 to 30, in particular of 8 to 29, in particular of 9 to 28, in particular of 10 to 27, in particular of 11 to 26, in particular of 12 to 25, in particular of 13 to 24, in particular of 14 to 23, in particular of 15 to 22, in particular of 16 to 21, in particular of 17 to 20, in particular of 18 or 19, amino acid residues. The amino acid residues may be amino acid residues which are positively charged at pH 7.4. The amino acid residues which are positively charged at pH 7.4 may be consecutive amino acid residues.

The number and sequence of amino acid residues positively charged at pH 7.4 can vary. The positively charged amino acid residues may comprise or consist of histidine residue(s), arginine residue(s), lysine residue(s) and/or positively charged analog(s) of amino acid residue(s) and in particular only comprise or consist of arginine residue(s) and/or lysine residue(s). The amino acid residue(s) may be L-isomer(s) or D-isomer(s). The histidine residues(s) may be alpha-histidine or beta-histidine residue(s), the arginine residues(s) may be alpha-arginine or beta-arginine residue(s) and the lysine residues(s) may be alpha-lysine or beta-lysine residue(s). Different numbers and combinations of lysine residue(s), arginine residue(s), histidine residue(s), and positively charged analog(s) of amino acid residue(s) result in different condensing profiles and endosome/lysosome escaping ability. The positively charged analog of the amino acid residue may be an analog of an aspartic acid residue such as a residue of an N-substituteted aspartamide, d-aspartamide, or beta-aspartamide, or an analog of a glutamatic acid residue such as a residue of an N-substituteted glutamide, d-glutamide, or beta-glutamide. "N-substituteted" means that the carboxyl group on the side chain of aspartic acid residue or glutamic acid residue is modified with a chemical group containing primary, secondary and/or tertiary amine(s). For example, an N-substituteted aspartamide may be 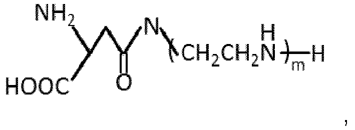 wherein m ≥ 1 and an N-substituteted glutamide may be 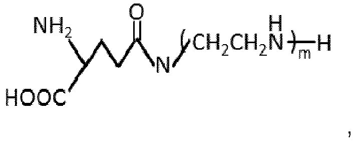 wherein m ≥ 1. The hydrophobic moiety can be covalently linked to any part of the peptide residue. In one embodiment of the invention the hydrophobic moiety is covalently linked to the amino terminus of the peptide residue or to the carboxy terminus of the peptide residue or to any side chain of any amino acid residue of the peptide residue, in particular the side chain of lysine, leucine, arginine, histidine, glutamic acid or aspartic acid. The further peptide residue may have a sequence with more than 40 %, in particular with more than 50 %, in particular with more than 60 %, amino acid residues with hydrophobic side chains. Side chains of amino acids are considered as hydrophobic if the hydrophobicity index at pH 2 and pH 7 according to the following table disclosed in www.sigmaaldrich.com is above 40:

| At pH 2* | | At pH 7** | |
|---|---|---|---|
| Very Hydrophobic | | | |
| Leucine (leu) | 100 | Phenylalanine (phe) | 100 |
| Isoleucine (ile) | 100 | Isoleucine (ile) | 99 |
| Phenylalanine (phe) | 92 | Tryptophan (trp) | 97 |
| Tryptophan (trp) | 84 | Leucine (leu) | 97 |
| Valine (val) | 79 | Valine (val) | 76 |
| Methionine (met) | 74 | Methionine (met) | 74 |

| Hydrophobic | | | |
|---|---|---|---|
| Cysteine (cys) | 52 | Tyrosine (tyr) | 63 |
| Tyrosine (tyr) | 49 | Cysteine (cys) | 49 |
| Alanine (ala) | 47 | Alanine (ala) | 41 |

| Neutral | | | |
|---|---|---|---|
| Threonine (thr) | 13 | Threonine (thr) | 13 |
| Glutamic acid (glu) | 8 | Histidine (his) | 8 |
| Glycine (gly) | 0 | Glycine (gly) | 0 |
| Serine (ser) | -7 | Serine (ser) | -5 |
| Glutamine (gln) | -18 | Glutamine (gln) | -10 |
| Aspartic acid (asp) | -18 | | |

| Hydrophilic | | | |
|---|---|---|---|
| Arginine (arg) | -26 | Arginine (arg) | -14 |
| Lysine (lys) | -37 | Lysine (lys) | -23 |
| Asparagine (asn) | -41 | Asparagine (asn) | -28 |
| Histidine (his) | -42 | Glutamic acid (glu) | -31 |
| Proline (pro) | -46 | Proline (pro) | -46 (used pH 2) |
| | | Aspartic acid (asp) | -55 |

| | | | |
|---|---|---|---|
| * pH 2 values: Normalized from Sereda et al., J. Chrom. 676: 139-153 (1994). ** pH 7 values: Monera et al., J. Protein Sci. 1: 319-329 (1995). | | | |

The hydrophobicity index indicates relative hydrophobicity and therewith how soluble an amino acid is in water. Values in the table are normalized by giving the most hydrophobic residue a value of 100 relative to glycine, which is considered neutral (0 value). The scales were extrapolated to residues which are more hydrophilic than glycine.

Amino acid residues with hydrophobic side chains are in particular amino acid residues of leucine, isoleucine, phenylalanine, tryptophan, valine, methionine, cysteine, tyrosine, alanine and their derivatives, in particular amino acid residues of phenylalanine, tyrosine and tryptophan.

An advantage of the lipoic acid residue is that lipoic acid is produced naturally in the human body and commonly used as an antioxidant drug for treating diseases such as diabetes and HIV.

Hydrophobicity and therewith efficiency of transfection can further be increased by amidation of the carboxy terminus of the peptide residue such that the C-terminus of the peptide residue is formed by a -CONH₂ moiety.

The lipoic acid residue and the lipoamide residue can firmly interact with the hydrophobic part of the amphiphilic block copolymer. The disulfide bond localized in the dithiolane of the lipoic acid residue and the lipoamide residue can be specifically cleaved by glutathione (GSH) which exists in high level in the cytoplasm of eukaryotic cells. As a result hydrophobicity of the lipoic acid residue or the lipoamide residue is decreased and its interaction with the amphiphilic block copolymer is weakened. This is followed by release of the synthetic compound from the amphiphilic block copolymer and an enhanced release of the protein or the polypeptide from the delivery system. As a result a higher delivery rate of the protein or the polypeptide is achieved.

The further peptide residue may consist of 5 to 32, In particular of 6 to 31, In particular of 7 to 30, in particular of 8 to 29, in particular of 9 to 28, in particular of 10 to 27, in particular of 11 to 26, in particular of 12 to 25, in particular of 13 to 24, in particular of 14 to 23, in particular of 15 to 22, in particular of 16 to 21, in particular of 17 to 20, in particular of 18 to 19, amino acid residues.

The targeting moiety may consist of 5 to 32, in particular of 6 to 31, in particular of 7 to 30, in particular of 8 to 29, in particular of 9 to 28, in particular of 10 to 27, in particular of 11 to 26, in particular of 12 to 25, in particular of 13 to 24, in particular of 14 to 23, in particular of 15 to 22, in particular of 16 to 21, in particular of 17 to 20, in particular of 18 to 19, amino acid residues.

The maximal length of the peptide residue, optionally together with the further peptide residue, may be 96, in particular 90, in particular 84, in particular 78, in particular 72, in particular 66, in particular 62, in particular 60, in particular 54, in particular 48, in particular 42, in particular 36, in particular 30, in particular 24, in particular 18, in particular 12, in particular 9, amino acid residues. The shorter the peptide residue, optionally together with the further peptide residue, is the lesser is the probability that it exerts an antigenic effect. This may be particular important if repeated administration is intended.

The peptide residue may consist of the protein binding or polypeptide binding moiety and optionally the targeting moiety. The peptide residue consisting of the protein binding or polypeptide binding moiety and the targeting moiety may comprise or consist of a polypeptide residue having a sequence consisting of any of SEQ ID NO. 52 to 73 or a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID NO. 52 to 73. The sequence similarity is calculated according to the EMBOSS needle algorithm having an open gap penalty of 10.0, an extended gap penalty of 0.5 and using the Blosum 62 matrix.

The peptide residue, optionally together with the further peptide residue, may comprise or consist of a polypeptide residue having a sequence consisting of any of SEQ ID NO. 74 to 103 or a sequence having a sequence similarity of at least 90 %, in particular of at least 91 %, in particular of at least 92 %, in particular of at least 93 %, in particular of at least 94 %, in particular of at least 95 %, in particular of at least 96 %, in particular of at least 97 %, in particular of at least 98 %, in particular of at least 99 %, to any of SEQ ID NO. 74 to 103. The sequence similarity is calculated according to the EMBOSS needle algorithm having an open gap penalty of 10.0, an extended gap penalty of 0.5 and using the Blosum 62 matrix.

In SEQ ID NO. 4, 95, 111, 112, amino acid residue X is 6-aminohexanoic acid and amino acid residue A is β-alanine.

The protein or the polypeptide may be a protein or a polypeptide having a molecular weight in a range of 2 kDa to 300 kDa, in particular in a range of 5 kDa to 200 kDa, in particular in a range of 20 kDa to 180 kDa, in particular in a range of 35 kDa to 140 kDa, in particular in a range of 50 kDa to 100 kDa.

The protein or the polypeptide may comprise at least one peptide modification at the amino terminus and/or at the carboxy terminus, in particular an acetylation, cyclization, methylation or phosphorylation. The amino terminus or the carboxy terminus of the protein or the polypeptide may be covalently linked to the amino terminus or to the carboxy terminus of a further protein, a further oligopeptide or a further polypeptide directly or by means of any protein linking or peptide linking molecule. Alternatively, the amino terminus or the carboxy terminus of the protein or the polypeptide may be covalently linked to a carbohydrate, to an aptamer or to an immunologic adjuvant.

The protein linking molecule may be a GSG linker, a (GSG)₂ linker, a (GSG)₃ linker, a (GSG)₄ linker, a (GSG)₅ linker, a GSGS linker, a (GSGS)₂ linker, a (GSGS)₃ linker, a (GSGS)₄ linker, a (GSGS)₅ linker, a GGG linker, a GGGG linker, a (GGG)₂ linker, a (GGGG)₂ linker, a (GGGGG)₂ linker, a GGGGS linker, a (GGGGS)₂ linker, a (GGGGS)₃ linker, an H3 linker, an H4 linker, an H6 linker, an H8 linker, an EAAK linker, an (EAAK)₂ linker, an (EAAK)₃ linker, or a combination of at least two of a GSG linker, a (GSG)₂ linker, a (GSG)₃ linker, a (GSG)₄ linker, a (GSG)₅ linker, a GSGS linker, a (GSGS)₂ linker, a (GSGS)₃ linker, a (GSGS)₄ linker, a (GSGS)₅ linker, a GGG linker, a GGGG linker, a (GGG)₂ linker, a (GGGG)₂ linker, a (GGGGG)₂ linker, a GGGGS linker, a (GGGGS)₂ linker, a (GGGGS)₃ linker, an H3 linker, an H4 linker, an H6 linker, an H8 linker, an EAAK linker, an (EAAK)₂ linker and an (EAAK)₃ linker. The peptide linking molecule may be a GSG linker, a (GSG)₂ linker, a (GSG)₃ linker, a (GSG)₄ linker, a (GSG)₅ linker, a GSGS linker, a (GSGS)₂ linker, a (GSGS)₃ linker, a (GSGS)₄ linker, a (GSGS)₅ linker, a GGG linker, a GGGG linker, a (GGG)₂ linker, a (GGGG)₂ linker, a (GGGGG)₂ linker, a GGGGS linker, a (GGGGS)₂ linker, a (GGGGS)₃ linker, an H3 linker, an H4 linker, an H6 linker, an H8 linker, an EAAK linker, an (EAAK)₂ linker, an (EAAK)₃ linker, or a combination of at least two of a GSG linker, a (GSG)₂ linker, a (GSG)₃ linker, a (GSG)₄ linker, a (GSG)₅ linker, a GSGS linker, a (GSGS)₂ linker, a (GSGS)₃ linker, a (GSGS)₄ linker, a (GSGS)₅ linker, a GGG linker, a GGGG linker, a (GGG)₂ linker, a (GGGG)₂ linker, a (GGGGG)₂ linker, a GGGGS linker, a (GGGGS)₂ linker, a (GGGGS)₃ linker, an H3 linker, an H4 linker, an H6 linker, an H8 linker, an EAAK linker, an (EAAK)₂ linker and an (EAAK)₃ linker. The protein linking molecule or peptide linking molecule corresponds to the following SEQ ID NOs. in the sequence listing:

| **Protein linking molecules or peptide linking molecules** | |
|---|---|
| GSGGSG | SEQ ID NO. 146 |
| GSGGSGGSG | SEQ ID NO. 147 |
| GSGGSGGSGGSG | SEQ ID NO. 148 |
| GSGGSGGSGGSGGSG | SEQ ID NO. 149 |
| GSGS | SEQ ID NO. 150 |
| GSGSGSGS | SEQ ID NO. 151 |
| GSGSGSGSGSGS | SEQ ID NO. 152 |
| GSGSGSGSGSGSGSGS | SEQ ID NO. 153 |
| GSGSGSGSGSGSGSGSGSGS | SEQ ID NO. 154 |
| GGGG | SEQ ID NO. 155 |
| GGGGGG | SEQ ID NO. 156 |
| GGGGGGGG | SEQ ID NO. 157 |
| GGGGGGGGGG | SEQ ID NO. 158 |
| GGGGS | SEQ ID NO. 159 |
| GGGGSGGGGS | SEQ ID NO. 160 |
| GGGGSGGGGSGGGGS | SEQ ID NO. 161 |
| HHHH | SEQ ID NO. 162 |
| HHHHHH | SEQ ID NO. 137 |
| HHHHHHHH | SEQ ID NO. 163 |
| EAAK | SEQ ID NO. 164 |
| EAAKEAAK | SEQ ID NO. 165 |
| EAAKEAAKEAAK | SEQ ID NO. 166 |

The further protein may be the *Eschericia coli* heat labile enterotoxin (LT), the *Escherichia coli* heat labile enterotoxin B subunit (LTB), the cholera toxin (CT), the cholera toxin B subunit (CTB) or an antibody. The antibody may be specifically binding to the protein binding or polypeptide binding moiety, in particular the His tag(s), the FLAG tag(s), the HA tag(s), the myc tag(s), the V5 tag(s), the Avi tag(s), the Strep-tag(s) II, the S tag(s) or the E tag(s). The inventors found that by covalently linking the protein or the polypeptide to a further protein, in particular to LTB, the immunogenicity of the protein or the polypeptide is increased.

The further oligopeptide or the further polypeptide may be a fragment of the further protein, in particular an antibody fragment specifically binding to the protein binding or polypeptide binding moiety, in particular the His tag(s), the FLAG tag(s), the HA tag(s), the myc tag(s), the V5 tag(s), the Avi tag(s), the Strep-tag(s) II, the S tag(s) or the E tag(s).

The protein to be delivered may be ovalbumin, an antigen, in particular a surface antigen, or an antibody, in particular a therapeutic antibody. The antigen may be an antigen of a virus, an antigen of a pathogen, in particular an antigen of a pathogenic microorganism, in particular an antigen of a pathogenic bacterium, an antigen specifically expressed by a cell affected by a disease, in particular an antigen specifically expressed by a cell affected by cancer, an antigen specifically expressed by a cell infected by a virus or an antigen specifically expressed by a cell infected by a pathogen, in particular an antigen specifically expressed by a cell infected by a pathogenic microorganism, in particular an antigen specifically expressed by a cell infected by a pathogenic bacterium. The antigen may be in particular the receptor binding domain (RBD) within the spike protein of SARS-CoV-2 virus, the Pseudomonas aeruginosa subunit antigen (PcrV) and LTB. The antibody may be an antibody specifically binding to an antigen of a virus, an antibody specifically binding to an antigen of a pathogen, an antibody specifically binding to an antigen of a pathogenic microorganism, in particular an antibody specifically binding to an antigen of a pathogenic bacterium, or an antibody specifically binding to an antigen specifically expressed by a cell affected by a disease, in particular an antibody specifically binding to an antigen specifically expressed by a cell affected by cancer. The antibody may specifically bind to the RBD within the spike protein of SARS-CoV-2 virus, the hemagglutinin (HA) of influenza, the neuraminidase (NA) of influenza, the PcrV, the LTB, the programmed cell death protein 1 (PD-1) or the cytotoxic T-lymphocyte-associated protein 4 (CTLA-4).

The polypeptide to be delivered may be a peptide hormone, In particular insulin, or an immunostimulant, in particular thymopentin.

The protein to be delivered may be a cationic protein or an anionic protein. The polypeptide to be delivered may be a cationic polypeptide or an anionic polypeptide. The terms "cationic protein" or "cationic polypeptide" refer to a protein or polypeptide with an electrostatic charge of greater than zero at pH 7.4 in aqueous solution, respectively. The terms "anionic protein" or "anionic polypeptide" refer to a protein or a polypeptide with an electrostatic charge of less than zero at pH 7.4 in aqueous solution, respectively. The electrostatic charge of the protein or the polypeptide to be delivered can be determined for example by zeta potential measurements of the protein or the polypeptide to be delivered in physiological solutions or by computational analysis of electrostatics of the protein or the polypeptide to be delivered.

In order for the ternary complex of the composition according to the invention to be formed, the protein binding or polypeptide binding moiety and the protein or the polypeptide to be delivered must bind to each other, either non-specifically, for example via ionic interactions, or by specifically binding to each other. Accordingly, the person skilled in the art would select the protein binding or polypeptide binding moiety of the synthetic compound depending on the protein or polypeptide to be delivered. For example, if a ternary complex containing an anionic protein to be delivered is to be formed, the person skilled in the art would select a cationic moiety as protein binding or polypeptide binding moiety. Accordingly, if for example a ternary complex containing a cationic polypeptide to be delivered is to be formed, the person skilled in the art would select an anionic moiety as protein binding or polypeptide binding moiety. If the amino acid sequence of the protein or polypeptide is known, the electrostatic charge of the protein or polypeptide at physiological pH, i. e. pH 7.4, can be determined, for example, by computational analysis of the protein or polypeptide. If the sequence of the protein or polypeptide is not known, the electrostatic charge of the protein or polypeptide can be determined, for example, by zeta potential measurements in physiological solutions of the protein or polypeptide.

If the protein or the polypeptide to be delivered is neither a cationic protein or polypeptide, nor an anionic protein or polypeptide, the person skilled in the art would select a protein binding or polypeptide binding moiety that binds specifically to a moiety of the protein or the polypeptide. In this case, the person skilled in the art would select for example a His tag as protein binding or polypeptide binding moiety which specifically binds to a protein or a polypeptide comprising an antibody or an antibody fragment thereof specifically binding to the His tag.

Thus, in order for the ternary complex to be formed, the person skilled in the art is able of selecting protein or polypeptide binding moieties that bind to the protein or polypeptide to be delivered by routine experimental work comprising only routine trials.

The amphiphilic block copolymer may be a poloxamer, in particular poloxamer 84, poloxamer 101, poloxamer 105, poloxamer 122, poloxamer 123, poloxamer 124, poloxamer 181, poloxamer 182, poloxamer 184 (designated L64), poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 304, poloxamer 331, poloxamer 333, poloxamer 334, poloxamer 335, poloxamer 338, poloxamer 401, poloxamer 402, poloxamer 403, poloxamer 407, a mixture of at least two of poloxamer 84, poloxamer 101, poloxamer 105, poloxamer 122, poloxamer 123, poloxamer 124, poloxamer 181, poloxamer 182, poloxamer 184, poloxamer 185, poloxamer 188, poloxamer 212, poloxamer 215, poloxamer 217, poloxamer 231, poloxamer 234, poloxamer 235, poloxamer 237, poloxamer 238, poloxamer 282, poloxamer 284, poloxamer 288, poloxamer 304, poloxamer 331, poloxamer 333, poloxamer 334, poloxamer 335, poloxamer 338, poloxamer 401, poloxamer 402, poloxamer 403 and poloxamer 407, or a poloxamine, in particular an ethylene oxide - propylene oxide copolymer with tetraether with (1,2-ethanedinitrilo)tetrakis(propanol), in particular poloxamine 304 (designated T304), poloxamine 701, poloxamine 704 (designated T704), poloxamine 901, poloxamine 904 (designated T904), poloxamine 908, poloxamine 1107, poloxamine 1301, poloxamine 1304, poloxamine 1307, poloxamine 90R4 (designated T90R4), poloxamine 150R1 or a mixture of at least two of poloxamine 304 (designated T304), poloxamine 701, poloxamine 704 (designated T704), poloxamine 901, poloxamine 904 (designated T904), poloxamine 908, poloxamine 1107, poloxamine 1301, poloxamine 1304, poloxamine 1307, poloxamine 90R4 (designated T90R4) and poloxamine 150R1.

The amphiphilic lipopeptide may be Pam₃-CSKKKK, PHC-SKKKK, Pam₂Cys-SKKKK, PamDhc-SKKKK or FSL-1.

Examples of amphiphilic lipopeptides are as follows: 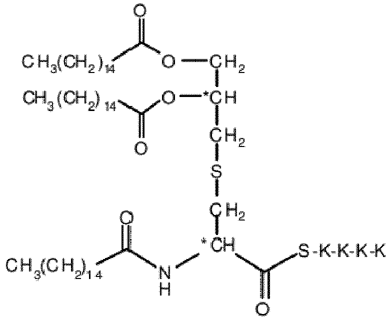 Pam₃Cys-SKKKK 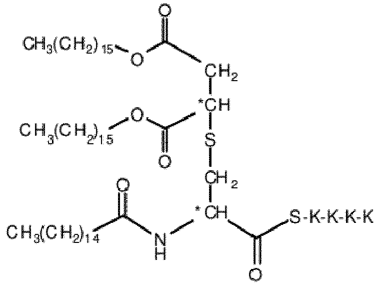 PHC-SKKKK 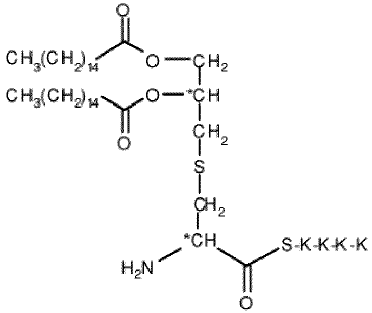 Pam₂Cys-SKKKK 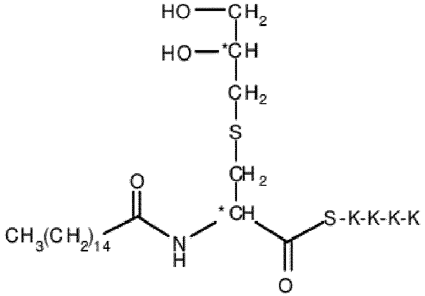 Pam-Dhc-SKKKK 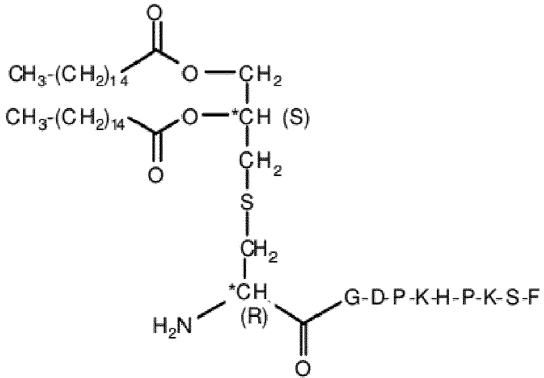 FSL-1

The amphiphilic block copolymer and the amphiphilic lipopeptide are amphiphiles. Amphiphiles are molecules comprising at least one hydrophobic domain and at least one hydrophilic domain, or may be molecules consisting of one, two or three hydrophobic domains and one, two or three hydrophilic domains. The amphiphilic block copolymer, in particular the hydrophobic domain of the amphiphilic block copolymer, or the amphiphilic lipopeptide, in particular the hydrophobic domain of the amphiphilic block copolymer, binds to the hydrophobic moiety of the synthetic compound. The inventors of the present invention found that the ternary complex consisting of the synthetic compound, either the amphiphilic block copolymer or the amphiphilic lipopeptide, and either the protein or the polypeptide may comprise an outer region of the ternary complex and an inner region of the ternary complex if the ternary complex as specified according to the invention is formed in an aqueous buffer solution. The inventors of the present invention further found that the hydrophobic domain of the amphiphilic block copolymer or the hydrophobic domain of the amphiphilic lipopeptide and the hydrophobic moiety of the synthetic compound may be present in the inner region of the ternary complex. The inventors of the present invention further found that the hydrophilic domain of the amphiphilic block copolymer or the hydrophilic domain of the amphiphilic lipopeptide and the targeting moiety of the synthetic compound may be present in the outer region of the ternary complex. Furthermore, the inventors of the present invention found that the protein or the polypeptide and the protein binding or polypeptide binding moiety of the synthetic compound may be present between the inner region and the outer region of the ternary complex.

The composition may further comprise an immunologic adjuvant, wherein the immunologic adjuvant may be selected from a group comprising or consisting of CpG oligodeoxynucleotides, polyinosinic:polycytidylic acid, *Quillaja saponaria* extract (QS-21 extract), monophosphoryl-lipid A, IL-2, IL-12, granulocyte-macrophage colony-stimulating factor (GM-CSF), thymic stromal lymphopoietin (TSLP), cyclic di-adenosine monophosphate (c-di-AMP), cyclic guanosine monophosphate-adenosine monophosphate (cGAMP), an aluminum salt, a zinc salt, a manganese salt, squalene, lipopolysaccharide (LPS), alpha-tocopherol, a polysorbate, a sorbitan ester, a lipid positively charged at pH 7.4, an ionizable lipid, a zwitterionic lipid or flagellin. The ionizable lipid is a lipid molecule remaining neutral at physiological pH, but is protonated at a relatively low pH, in particular at a pH below 5, in particular at a pH below 4, in particular at a pH below 3, in particular at a pH below 2. The lipid positively charged at pH 7.4 may be dioleoyl-3-trimethylammonium propane (DOTAP), 1,2-di-O-octadecenyl-3-trimethylammonium propane (DOTMA), 2,3- dioleyloxy-N-[2-(sperminecarboxamido)ethyl]-N,N-dimethyl-1-propanaminium trifluoroacetate (DOSPA), ethylphosphatidylcholine (ePC), N⁴-cholesteryl-spermine (GL67) or imidazole cholesterol ester (ICE). The ionizable lipid may be (6Z,9Z,28Z,31Z)-heptatriacont-6,9,28,31-tetraene-19-yl 4-(dimethylamino)butanoate (DLin-MC3-DMA), [(4-hydroxybutyl)azanediyl]di(hexane-6,1-diyl) bis(2-hexyldecanoate) (ALC-0315),heptadecan-9-yl 8-((2-hydroxyethyl) (6-oxo-6-(undecyloxy)hexyl)amino) octanoate (Lipid H, SM-102), tetrakis(8-methylnonyl) 3,3',3",3‴-(((methylazanediyl)*bis*(propane-3,1-diyl))*bis*(azanetriyl))tetrapropionate (3060ᵢ₁₀), 2-(dioctylamino)ethyl nonyl hydrogen phosphate (9A1P9), ethyl 5,5-di((Z)-heptadec-8-en-1-yl)-1-(3-(pyrrolidin-1-yl)propyl)-2,5-dihydro-1H-imidazole-2-carboxylate (A2-Iso5-2DC18), bis(2-(dodecyldisulfanyl)ethyl) 3,3'-((3-methyl-9-oxo-10-oxa-13,14-dithia-3,6-diazahexacosyl)azanediyl) dipropionate (BAME-016B), 1,1'-((2-(4-(2-((2-(bis(2-hydroxydodecyl) amino)ethyl) (2-hydroxydodecyl)amino)ethyl) piperazin-1-yl)ethyl)azanediyl) bis(dodecan-2-ol) (C12-200), 3,6-bis(4-(bis(2-hydroxydodecyl)amino)butyl)piperazine-2,5-dione (cKK-E12), (((3,6-dioxopiperazine-2, 5-diyl)bis(butane-4, 1-diyl))bis(azanetriyl))tetrakis(ethane-2,1-diyl) (9Z,9'Z,9"Z,9‴Z, 12Z,12'Z,12"Z,12‴Z)-tetrakis (octadeca-9,12-dienoate) (OF-Deg-Lin), N1,N3,N5-tris(3-(didodecylamino)propyl)benzene-1,3,5-tricarboxamide (TT3), hexa(octan-3-yl) 9,9',9",9‴,9ʺʺ,9‴ʺ- ((((benzene-1,3,5-tricarbonyl)yris(azanediyl)) tris (propane-3,1-diyl)) tris(azanetriyl))hexanonanoate (FTT5), C12 lipid coupled 2-3-2 oligoalkylamine (C12-(2-3-2)), 9-[4-(dimethylamino)-1-oxobutoxy]-heptadecanedioic acid, 1,17-di-(2Z)-2-nonen-1-yl ester (L319), N,N-dimethyl-2,2-di-(9Z,12Z)-9,12-octadecadien-1-yl-1 ,3-dioxolane-4-ethanamine (DLin-KC2-DMA), 1,2-Dilinoleyloxy-N,N-dimethyl-3-aminopropane (DLinDMA), 8-[(2-hydroxyethyl)[8-(nonyloxy)-8-oxooctyl]amino]-octanoic acid 1-octylnonyl ester (Lipid 5) or L343 (CAS. Nr. 1443520-22-6). The zwitterionic lipid may be cephalin. The immunologic adjuvant may improve an immune response directed against the protein or the polypeptide, in particular if the protein or the polypeptide is delivered to an epithelial cell, in particular to a respiratory epithelial cell, or to an immune cell, in particular to a dendritic cell, to a macrophage, to a T cell or to a B cell.

Examples of lipids positively charged at pH 7.4 (cationic lipids) are as follows:

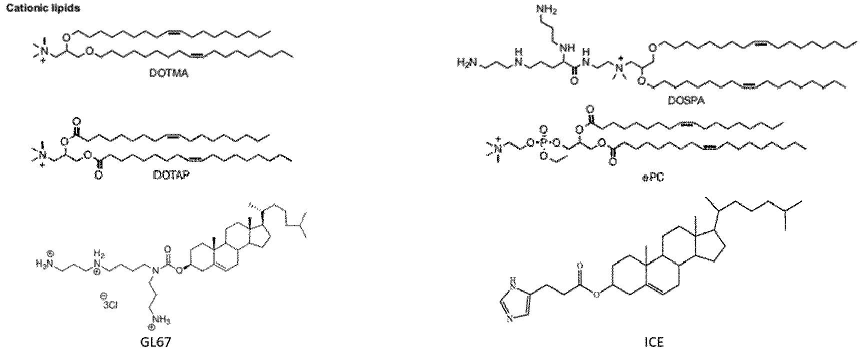

Examples of ionizable lipids are as follows:

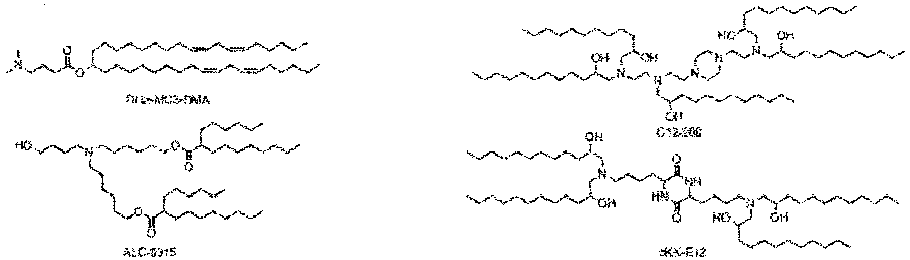

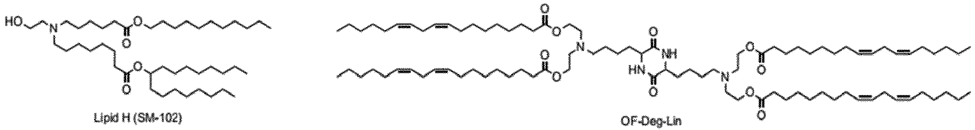

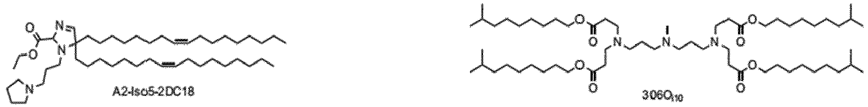

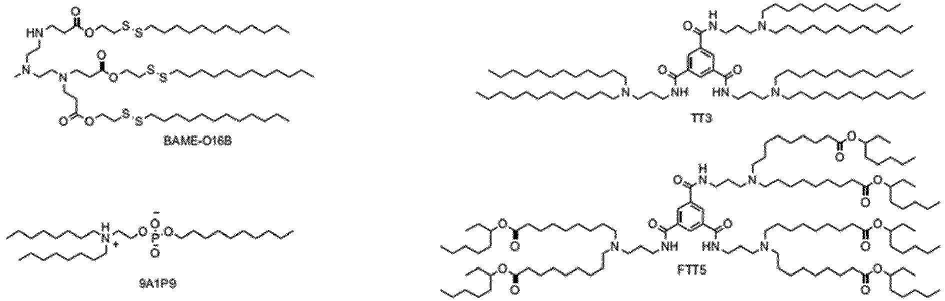

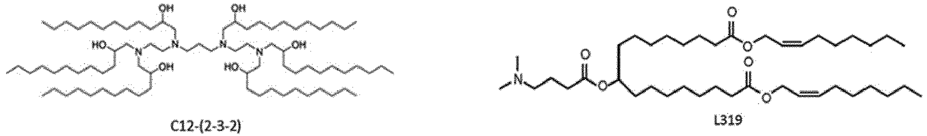

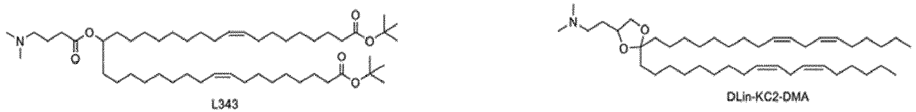

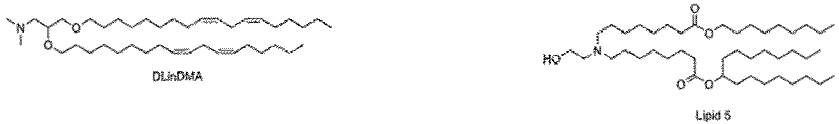

The composition may further comprise a nucleic acid. The nucleic acid may be an RNA, in particular an mRNA, in particular an *in vitro* transcribed messenger RNA (IVT-mRNA), or an siRNA, or a DNA and it may be present as a plasmid (pDNA or pRNA), a single strand or a double strand.

The invention also concerns the composition according to the invention for use as a medicament. The medicament may be a medicament for the treatment of a disease of a human being or an animal by recombinant protein therapy or by peptide therapy or by protein replacement therapy, in particular by enzyme replacement therapy, or by an immunotherapy or a medicament for preventing an infection by vaccination of a human being or an animal. The composition according to the invention can be present dissolved in a solution or suspension, in particular a nebulized solution or suspension, or as a dry powder formulation. The medicament may be administered by injection, by nasal administration, by inhalation, by oral administration, by dermal administration, by intravaginal administration or by rectal administration. The animal may be a mammal. The disease may be a genetically caused disease or an acquired disease. The genetically caused disease may be cancer, type I diabetes, cystic fibrosis, hemophilia or muscular dystrophy. The acquired disease may be cancer, type II diabetes, a disease acquired by viral infection or a disease acquired by infection with a pathogenic microorganism, in particular by infection with a pathogenic bacterium. In case of an immunotherapy of a genetically caused disease, such as a cancer, the protein or the polypeptide may be a specific antigen or an immunodominant epitope of a specific antigen, in particular a surface antigen, specifically expressed by cells affected by a genetic alteration causing the genetically caused disease. In case of use in preventing an infection by vaccination the infection may be an infection by a virus or a pathogen such as a pathogenic microorganism, in particular a pathogenic bacterium. In this case the protein or the polypeptide may be a specific antigen or an immunodominant epitope of a specific antigen, in particular a surface antigen, specifically expressed by cells infected with the virus or the pathogen or a surface antigen of the virus or the pathogen. In both cases, the immunotherapy and the prevention of an infection by vaccination, the delivery of the protein or the polypeptide results in an immune response of the treated human being or animal. The immune response may be specific for the protein or the polypeptide. In cases of the immunotherapy or the vaccination, the composition according to the invention may be injected in the human being or animal, in particular by subcutaneous injection, by intramuscular injection, by intratumoral injection, by intracerebral injection, by intradermal injection, by intraperitoneal injection, by intranasal injection, by intratracheal injection or by intravenous injection, or inhaled by the human being or animal, in particular by inhalation of the composition according to the invention in a nebulized form or as dry powder formulation.

The composition may further comprise a pharmaceutically acceptable carrier. The pharmaceutically acceptable carrier may comprise or consist of water or a salt solution, in particular a physiologic salt solution, a phosphate-buffered saline (PBS) solution, a 4-(2-hydroxyethyl)-1-piperazineethanesulfonic acid (HEPES) solution, a tris(hydroxymethyl)aminomethane (TRIS) solution, a Tyrode's solution, an Eagle's Minimum Essential Medium such as Opti-MEM^{®} (purchased from ThermoFisher Scientific), a sodium chloride solution or a calcium chloride solution. The composition, optionally further comprising the pharmaceutically acceptable carrier, may also be prepared as a dry powder formulation. In this case the pharmaceutically acceptable carrier can comprise or consist of an albumin, a polyethylene glycol (PEG), or a saccharide such as trehalose, mannose or mannitol. The composition, optionally further comprising the pharmaceutically acceptable carrier, may be a pharmaceutical composition for use as a medicament. The medicament may be a medicament for the treatment of a disease of a human being or an animal by recombinant protein therapy or by peptide therapy or by protein replacement therapy, in particular by enzyme replacement therapy, or by an immunotherapy or a pharmaceutical composition for preventing an infection by vaccination.

The ternary complex as specified according to the invention may be taken up by any eukaryotic cell. The inventors found that the cellular uptake of the ternary complex is relatively low at a temperature of 4 °C, which renders the energy metabolism of a cell at a low level. Furthermore, the cellular uptake of the ternary complex is relatively low after adenosine triphosphate depletion by sodium azide and in the presence of the macropinocytosis inhibitor amiloride, the adsorptive-mediated endocytosis inhibitor protamine or the clathrin-mediated endocytosis inhibitor chlorpromazine. Furthermore, the cellular uptake of the ternary complex remains relatively high in the presence of the caveolae-mediated endocytosis inhibitor filipin or the proteoglycan sulfation inhibitor sodium chlorate. This indicates that the cellular uptake of the ternary complex may be mediated by endocytosis, in particular macropinocytosis, electrostatic absorptive endocytosis and clathrin-mediated endocytosis. In contrast to the ternary complex, the cellular uptake of a complex comprising the amphiphilic block copolymer and the protein or the polypeptide, but not the synthetic compound as specified according to the invention, is relatively low in general.

The inventors of the present invention further found that the cellular uptake mediated by the ternary complex as specified according to the invention can be directional, if the peptide residue optionally further comprises at least one targeting moiety. Since the optional targeting moiety is a moiety that causes a direction of the synthetic compound to a position or a structure inside or on the surface of the eukaryotic cell, the ternary complex containing the synthetic compound as specified according to the invention can be brought into close proximity to the eukaryotic cell the targeting moiety is specific for. After being brought into close proximity with the eukaryotic cell, the cellular uptake of the ternary complex may be mediated by endocytosis, in particular macropinocytosis, electrostatic absorptive endocytosis or clathrin-mediated endocytosis. This results in a directional cellular uptake of the ternary complex into the eukaryotic cells the targeting moiety is specific for.

The cellular uptake of the ternary complex by the eukaryotic cells may be mediated by endocytosis. Endocytosis is a process of translocation of a material to be internalized, in particular an extracellular material to be internalized, into cells. The material to be internalized is surrounded by an area of the cell membrane of the eukaryotic cell. The area of the cell membrane of the eukaryotic cell then buds off inside the cell to form a vesicle containing the material to be internalized. Endocytosis is a form of active transport and includes clathrin-mediated endocytosis (also known as receptor-mediated endocytosis), caveolae, pinocytosis and phagocytosis. Endocytosis is a fundamental cellular process in all eukaryotic cells. Since all eukaryotic cells can perform endocytosis, the ternary complex as specified according to the invention may be taken up by any eukaryotic cell. The eukaryotic cell may be an epithelium cell, in particular a respiratory epithelium cell or a microfold cell, an immune cell, in particular a macrophage, a dendritic cell, a T cell or a B cell, an endothelial cell, in particular a lung endothelial cell or a liver endothelial cell, and/or a skin cell, in particular a dermal fibroblast or a keratinocyte.

The invention further concerns the use of the composition according to the invention for *in vitro* delivery of the protein or the polypeptide to a eukaryotic cell in cell culture.

The invention also concerns a method for an *in vitro* delivery of a protein or a polypeptide to a eukaryotic cell in cell culture comprising the following steps:
a1) providing the synthetic compound, providing the protein or the polypeptide, providing the amphiphilic block copolymer or the amphiphilic lipopeptide and providing an aqueous buffer solution,
b1.1) mixing the synthetic compound with the amphiphilic block copolymer or the amphiphilic lipopeptide in the aqueous buffer solution to allow the formation of a binary complex,
b1.2) mixing the binary complex with the protein or the polypeptide in the aqueous buffer solution to allow the formation of a ternary complex and
c1) contacting the ternary complex with the eukaryotic cell
   or
a2) providing the synthetic compound, providing the protein or the polypeptide, providing the amphiphilic block copolymer or the amphiphilic lipopeptide and providing an aqueous buffer solution,
b2.1) mixing the protein or the polypeptide with the amphiphilic block copolymer or the amphiphilic lipopeptide in the aqueous buffer solution to allow the formation of a binary complex,
b2.2) mixing the binary complex with the synthetic compound in the aqueous buffer solution to allow the formation of a ternary complex and
c2) contacting the ternary complex with the eukaryotic cell.

The aqueous buffer solution may be an aqueous salt solution, in particular a physiological salt solution, a phosphate-buffered saline (PBS) solution, a 4-(2-hydroxyethyl)-1-piperazineethanesulfonic acid (HEPES) solution, a tris(hydroxymethyl)aminomethane (TRIS) solution, a Tyrode's solution, an Eagle's Minimum Essential Medium such as Opti-MEM^{®} (purchased from ThermoFisher Scientific), a sodium chloride solution or a calcium chloride solution. The pH value of the aqueous buffer solution may be in the range of 7.0 to 7.5 and in particular may be 7.4.

All features indicated in the specification are to be understood as features applicable to all embodiments of the invention. This means, for example, that a feature indicated for the composition according to the invention can also be applied to the use according to the invention and/or the method for an *in vitro* delivery of a protein or a polypeptide to a eukaryotic cell according to the invention, and vice versa. In the following, as far as not specified the term "average" always means "arithmetic average". As far as not specified the term "diameter" always means "Volume Equivalent Spherical Diameter" (=VESD). The particle size for determining that diameter can be determined by static light scattering, laser diffraction, microscopy, in particular optical microscopy, followed by image analysis, sedimentation analysis and by use of a disc centrifuge.

The invention is further illustrated on basis of the following embodiments.
- Fig. 1: shows the influence of the solvent system on the arithmetic average particle diameter of OVA-containing T704 based ternary complexes comprising compound 2,
- Fig. 2: shows the influence of the preparation method on the arithmetic average particle diameter of OVA-containing T704 based ternary complexes comprising compound 2,
- Fig. 3: shows the cellular uptake of an OVA-containing T90R4 based ternary complex comprising compound 2,
- Fig. 4: shows the elicited OVA-specific IgG antibodies in mouse serum samples after an intratracheal prime-boost immunization regimen comprising OVA-containing ternary complexes,
- Fig. 5: shows the elicited OVA-specific IgA antibodies in mouse vaginal lavage fluid samples after an intratracheal prime-boost immunization regimen comprising OVA-containing ternary complexes,
- Fig. 6: shows the elicited RBD-specific IgG antibodies in mouse serum samples after an intratracheal prime-boost immunization regimen comprising RBD-containing ternary complexes and LTB-RBD-containing ternary complexes,
- Fig. 7: shows the elicited RBD-specific IgA antibodies in mouse vaginal lavage fluid samples after an intratracheal prime-boost immunization regimen comprising RBD-containing ternary complexes and LTB-RBD-containing ternary complexes,
- Fig. 8: shows the elicited RBD-specific IgA antibodies in mouse bronchoalveolar lavage fluid samples after an intratracheal prime-boost immunization regimen comprising RBD-containing ternary complexes and LTB-RBD-containing ternary complexes,
- Fig. 9: shows the pseudovirus neutralization of SARS-CoV-2 by mouse bronchoalveolar lavage fluid samples and mouse serum samples after an intratracheal prime-boost immunization regimen comprising RBD-containing ternary complexes and LTB-RBD-containing ternary complexes,
- Fig. 10: shows the anti-SARS-CoV-2 pseudovirus neutralizing antibody titer in mouse serum samples and BALF samples after an intranasal prime-boost immunization regimen with an LTB-RBD-containing T90R4 based ternary complex containing compound 2,
- Figs. 11a and 11b: shows the influence of ternary complex dosage and route of administration on elicited RBD-specific IgG antibodies in mouse serum samples after a prime-boost immunization regimen comprising RBD-containing ternary complexes and LTB-RBD-containing ternary complexes,
- Fig. 12: shows the influence of ternary complex dosage and route of administration on elicited RBD-specific IgA antibodies in mouse vaginal lavage fluid samples after a prime-boost immunization regimen comprising RBD-containing ternary complexes and LTB-RBD-containing ternary complexes,
- Fig. 13: shows the influence of ternary complex dosage and route of administration on elicited RBD-specific IgA antibodies in mouse lung tissue samples after a prime-boost immunization regimen comprising RBD-containing ternary complexes and LTB-RBD-containing ternary complexes,
- Figs. 14: shows the elicited RBD-specific IgG antibodies in mouse serum samples after an intranasal prime-boost immunization regimen comprising RBD-containing ternary complexes and LTB-RBD-containing ternary complexes,
- Fig. 15: shows the IFN-gamma, IL-4 and IL-17A spot-forming cells among pulmonary lymphocytes after stimulation with a pool of 14-mer overlapping peptides spanning the SARS-CoV-2 RBD protein,
- Fig. 16a to 16d: shows the elicited PcrV-specific IgG antibodies in mouse serum samples, the elicited PcrV-specific IgA antibodies in mouse vaginal lavage fluid samples and mouse lung tissue samples and the CD4+CD17+ T cell frequency in mouse lung tissue samples after an intranasal prime-boost immunization regimen comprising a PcrV-containing ternary complex,

### Materials and Methods:

Reagents: Tetrafunctional block copolymers 704 (T704) were kindly provided by In-Cell-Art (Nantes, France). Poloxamine 304 (T304) and poloxamine 904 (T904) were kindly provided by BASF GmbH (Ludwigshafen, Germany). Poloxamine 90R4, poloxamer 184 (L64) and poloxamer 407 were purchased from Sigma-Aldrich Chemie GmbH (Taufkirchen, Germany).

Synthesis of peptide based compounds: All compounds were manually synthesized by Fmoc solid-phase peptide synthesis method. Briefly, 2-Chlorotrityl resin was used as the solid phase for the peptide synthesis. N,N-diisopropylethylamine (DIPEA) was used as the base and 2-(6-chloro-1H-benzotriazole-1-yl)-1,1,3,3-tetramethylaminium hexafluorophosphate (HCTU) was served as the coupling reagent. Lipoic acid, myristic acid or cholesterol was coupled to the -NH₂ end of the peptides before cleaving the peptides from the resin and deprotection. The peptides were then cleaved from the resin by acetic acid/trifluoroethanol/DCM (v/v/v, 10/20/70) at room temperature for 2 h. The resin was removed by filtration and the solvent was evaporated. After deprotection in cocktails (TFA/phenol/water/TIPS, 88:5:5:2) at room temperature for 2 h, the solution was concentrated and added into ice-cold diethyl ether for precipitation. The final products were purified by reverse phase HPLC. The molecular weight was confirmed by LC/MS.

Unless otherwise indicated, the binary complexes containing the synthetic compound and the protein or the polypeptide were prepared as follows: Stock solutions of synthetic peptide (1.2 µg/µL) were prepared in 0.5 mM HEPES solution (pH 6.35) and stored at 4 °C until further use. The protein or polypeptide (1.2 µg/µL) was prepared in 0.5 mM HEPES solution (pH 6.35) and stored at -80 °C. The protein or the polypeptide (1.2 µg/µL) was gently mixed with a dilution of the synthetic peptide (0.3 µg/µL, diluted with 0.5 mM HEPES) with a v/v = 1:1. The final formulation was stored at 4 °C until further use.

Unless otherwise indicated, the ternary complexes containing the synthetic compound, the amphiphilic block copolymer or the amphiphilic lipopeptide and the protein or the polypeptide were prepared as follows: Stock solutions of the amphiphilic block copolymer or the amphiphilic lipopeptide (10.0 µg/µL) and stock solutions of the synthetic peptide (1.2 µg/µL) were prepared in 0.5 mM HEPES solution (pH 6.35). The stock solutions were stored at 4 °C until further use. The protein or polypeptide (1.2 µg/µL) was prepared in 0.5 mM HEPES solution (pH 6.35) and stored at -80 °C. The ternary complex was generated via a two-step incubation process. Briefly, the solution of the amphiphilic block copolymer or the amphiphilic lipopeptide at a specific concentration (e.g. 5 µg/µL) in 0.5 mM HEPES solution (pH 6.35) was mixed with a dilution of the protein or polypeptide at a specific concentration (e.g. 1.2 µg/µL) with a v/v = 2:1. After 20 min incubation at 4 °C, a dilution of the synthetic peptide at a specific concentration (e.g. 1.2 µg/µL) in 0.5 mM HEPES solution (pH 6.35) was added to the above mixture with a v/v = 1:3 and mixed gently by pipetting up and down several times. Subsequently, the ternary complex was incubated for 20 minutes at 4 °C before further use.

The following is an example of the preparation of a ternary complex containing poloxamer 407, compound 2 and OVA, as well as the immunologic adjuvant CpG: P407 stock solutions (20 % w/v) was prepared by dissolving P407 in 0.5 mM HEPES (pH 6.35) buffer under stirring on ice. The solution was kept in the fridge overnight to improve the dissolution. Stock solutions of the synthetic compound 2 (3.0 µg/µL) were prepared in 0.5 mM HEPES solution (pH 7.3) and stored at 4 °C. Poloxamer solution at a specific concentration (e.g. 20 % w/v) in 0.5 mM HEPES (pH 6.35) was mixed with protein solution at a specific concentration (e. g. 1.2 µg/µL) with a v:v = 2:1. Subsequently, CpG at a concentration of 0.167 µg/µL was added. After 20 minutes incubation on ice, the synthetic peptide at a specific concentration (e. g. 1.2 µg/µL) in 0.5 mM HEPES (pH 6.35) was added to the above copolymer/protein solution with a v:v = 1:3 and mixed gently by pipetting up and down several times. Subsequently, the ternary complex was incubated for another 20 min on ice before further use.

Measurement of the hydrodynamic average arithmetic particle diameter and polydispersity index of binary or ternary complexes: Dilute an aliquot of the binary complex solution or the ternary complex solution in NF water to obtain a final volume of 1 mL. Measure the hydrodynamic average arithmetic particle diameter and the polydispersity index (PDI) using a semi-micro cuvette. Add the diluted formulations into the cuvette and place it into the particle size meter (Malvern Zetasizer). Set up a standard operating procedure and click on Start to begin the data acquisition.

Mouse immunization: Female 8-10 week-old BALB/c mice or C57BL/6 mice were kept under specific pathogen-free conditions undergoing a 12 h/12 h light/dark cycle with free access to food and water. Animals were conceded an adaption time of at least 7 days before the beginning of the experiments. The mice were anaesthetized with a mixture of medetomidine (0.5 mg/kg), midazolam (5 mg/kg) and fentanyl (0.05 mg/kg), injected intraperitoneally. The mice were immunized on day 0 via intratracheal (i.t.) or intranasal (i.n.) route with ternary complexes containing antigen (e. g. 12 µg/mouse) in 50 µL solution (i.t. route) or 40 µL solution (i.n. route), and boosted with equal dose of the same formulation on day 14 and day 28 post prime immunization. The intratracheal spray application was performed using a high-pressure MicroSprayer device (Penn Century Inc., Wyndmoor USA)

Sample collection: Blood samples were collected on specific time-points after the prime immunization. The bronchoalveolar lavage fluids were collected by washing the lungs of euthanized mice with 700-1000 µl of ice-cold PBS containing 100 µm EDTA, 0.05 % sodium azide, 0.05 % Tween-20, and 1 × protease inhibitor (Pierce) (mucosal prep solutions (MPS) with a blunt-ended needle. The vaginal lavage fluids were obtained by rinsing with 75 µL of ice-cold PBS prepared as above described each times, four times a day. All the samples collected were centrifuged at 8000 x g for 10 min at 4 °C and the supernatants were stored at -80 °C till performing the ELISA assay.

Enzyme-linked immunosorbent assay: ELISA plates (Corning) were coated overnight with 2 µg/mL of SARS-CoV-2 RBD recombinant protein in 0.05 M carbonate-bicarbonate buffer, pH 9.6, and blocked in 5 % skimmed milk in PBS. Diluted serum or lavage fluid samples were added to each well. Plates were incubated with goat anti-mouse IgG-HRP or IgA-HRP antibodies and developed with 3,3',5,5'-tetramethylbenzidine (TMB) substrate. Reactions were stopped with 2 M hydrochloric acid, and the absorbance was measured at 450 nm using a microplate reader (PerkinElmer, USA). The endpoint titers were defined as the highest logarithmic dilution of serum to yield an absorbance greater than 2.1-fold of the background values.

Single cell suspension preparation: Pulmonary immune cells were collected by finely chopping harvested lung tissues, followed by digestion in a media containing 5 mg collagenase D (Thermo-Fisher Scientific) and 1.25 mg of DNase I (Thermo-Fisher Scientific) at 225 rpm in a shaker at 37 °C for 40 min.

Flow cytometry analyses, peptide restimulation and intracellular cytokine staining: Splenocytes from vaccinated mice were incubated in culture with a pool of SARS-CoV-2 RBD peptides for 12 h at 37 °C before a 4 h treatment with brefeldin A (BioLegend, 420601). Following blocking with FcgR antibody (BioLegend, clone 93), cells were stained on ice with CD3 (PE/Cyanine7) (BioLegend), CD4 (FITC) (BioLegend), and Fixable Aqua Dead Cell Stain (Invitrogen, L34966). Subsequently, intracellular staining was performed with the following cytokine antibody: anti-IL-17 (BD Biosciences, TC11-18H10)). Lungs from immunized mice were harvested and single cell suspension was prepared. Lung cells were incubated at 37 °C with the pool of SARS-CoV-2 RBD peptides described above for 8 h at 37 °C. Lung cells then were stained as described above. To evaluate the immune response and memory establishment, mice were primed at day 0 and boosted at day 14 and 28, respectively. Mice were sacrificed at day 70 for assessing memory immune response establishment, respectively, and relevant tissues were harvested and processed for flow cytometric analysis. All flow cytometry data were acquired on a BD LSRFortessa cytometer and were analyzed with FlowJo software.

The following synthetic compounds were synthesized:
1. Compound 1: KETWWETWWTEWWTEW**-KKKKRRRRRKKKK-***GACSERSMNFCG* (peptide sequence of this compound: SEQ ID NO. 75)
2. Compound 2: KETWWETWWTEWWTEW-LHHHKKLLHHHKKL-*GACSERSMNFCG* (peptide sequence of this compound: SEQ ID NO. 74)
3. Compound P0: KETWWETWWTEWWTEW**-KKKKRRRRRKKKK-***VKRKKKP* (peptide sequence of this compound: SEQ ID NO. 76)
4. Compound P9NLS: KETWWETWWTEWWTEW-**KKKKRRRRRKKKK-***GACSERSMNFCGVKRKKKP* (peptide sequence of this compound: SEQ ID NO. 77)
5. Compound P7: KETWWETWWTEWWTEW-**KKKKRRRRRKKKK-***GACYGLPHKFCG* (peptide sequence of this compound: SEQ ID NO. 78)
6. Compound P6: Lipoic acid-W-**KKKKRRRRRKKKK**-*GACSERSMNFCG* (peptide sequence of this compound: SEQ ID NO. 53)
7. Compound P11: C₁₃H₂₇CONH-**KKKKRRRRRKKKK**-*GACSERSMNFCG* (peptide sequence of this compound: SEQ ID NO. 53)
8. Compound P12: Cholesteryl-**KKKKRRRRRKKKK-***GACSERSMNFCG* (peptide sequence of this compound: SEQ ID NO. 53)
9. Compound P10: KETWWETWWTEWWTEW-**LHHHKKL-***GACSERSMNFCG* (peptide sequence of this compound: SEQ ID NO. 79)
10. Compound P8-H2: KETWWETWWTEWWTEW-**LHHHKKLLHHHKKLLHHHKKL**-*GACSERSMNFCG* (peptide sequence of this compound: SEQ ID NO. 80)
11. Compound P8: Lipoic acid-W**-LHHHKKLLHHHKKL-***GACSERSMNFCG* (peptide sequence of this compound: SEQ ID NO. 54)
12. Compound C2-NLS: KETWWETWWTEWWTEW-LHHHKKLLHHHKKL-*VKRKKKP* (peptide sequence of this compound: SEQ ID NO. 81)
13. Compound 3: *Ac*-*FAEKFKEAVKDYFAKFWDGSG*-**KKKKRRRRRKKKK-**ETWWETWWTEWWTEW (peptide sequence of this compound: SEQ ID NO. 82)
14. Compound 4: KETWWETWWTEWWTEW**-KKKKRRRRRKKKK-***FYPSYHSTPQRP* (peptide sequence of this compound: SEQ ID NO. 83)
15. Compound 5: KETWWETWWTEWWTEW-**KKKKRRRRRKKKK**-(*D-Mannose)* (peptide sequence of this compound: SEQ ID NO. 84)
16. Compound 6: KETWWETWWTEWWTEW**-LHHHKKLLHHHKKL-***GGGGGCYTYQGKLC* (peptide sequence of this compound: SEQ ID NO. 85)
17. Compound 7: KETWWETWWTEWWTEW-**LHHHKKLLHHHKKL-***GSGSGINNNLQRVRELAVQSANSTNSQSDLDS* (peptide sequence of this compound: SEQ ID NO. 86)
18. Compound 8: KETWWETWWTEWWTEW-**LHHHKKLLHHHKKL-**GSGSG*TITHFQFGPTVY* (peptide sequence of this compound: SEQ ID NO. 87)
19. Compound 9: KETWWETWWTEWWTEW**-LHHHKKLLHHHKKL-***GSGCKSTHPLSC* (peptide sequence of this compound: SEQ ID NO. 88)
20. Compound 10: KETWWETWWTEWWTEW-**LHHHKKLLHHHKKL-***GSGCRGDKGPDC* (peptide sequence of this compound: SEQ ID NO. 89)
21. Compound 11: KETWWETWWTEWWTEW-**LHHHKKLLHHHKKL-***GSGSLDAGQYVLVM* (peptide sequence of this compound: SEQ ID NO. 90)
22. Compound 12: KETWWETWWTEWWTEW**-LHHHKKLLHHHKKL-***KANSSYSGNYPYSILFQKF* (peptide sequence of this compound: SEQ ID NO. 91)
23. Compound 13: KETWWETWWTEWWTEW-**LHHHKKLLHHHKKL-***GSGKIAIVNVSSIFQQLPARET* (peptide sequence of this compound: SEQ ID NO. 92)
24. Compound 14: KETWWETWWTEWWTEW-**LHHHKKLLHHHKKL-***GSGSFHQLPARSPLP* (peptide sequence of this compound: SEQ ID NO. 93)
25. Compound 15: KETWWETWWTEWWTEW-**KLLLLKLLLLKLLLLKLLLLK** (peptide sequence of this compound: SEQ ID NO. 94)
26. Compound 16: KETWWETWWTEWWTEW-**RXRRARRXRYQFLIRXRARXRA** (peptide sequence of this compound: SEQ ID NO. 95; in this peptide sequence: X = 6-aminohexanoic acid, A = β-alanine)
27. Compound 17: KETWWETWWTEWWTEW**-GLFGAIAGFIENGWEGMIDG** (peptide sequence of this compound: SEQ ID NO. 96)
28. Compound 18: KETWWETWWTEWWTEW-**RQIKIWFQNRRMKWKK** (peptide sequence of this compound: SEQ ID NO. 97)
29. Compound 19: KETWWETWWTEWWTEW-**AGYLLGKLLOOLAAAALOOLL** (peptide sequence of this compound: SEQ ID NO. 98)
30. Compound 20: KETWWETWWTEWWTEW-**AGYLLGKINLKALAALAKKIL** (peptide sequence of this compound: SEQ ID NO. 99)
31. Compound 21: KETWWWETWWTEWWTEWWE-**ARLARALARALARHLARALARALRACEA** (peptide sequence of this compound: SEQ ID NO. 100)
32. Compound 22: KETWWETWWTEWWTEWWE-**AKLAKALAKALAKHLAKALAKALKACEA** (peptide sequence of this compound: SEQ ID NO. 101)
33. Compound 23: KETWWETWWTEWWTEWWE-**AGLAGALAGALAGHLAGALAGALGACEA** (peptide sequence of this compound: SEQ ID NO. 102)
34. Compound 24: VVVVVV-**KRRRRK**-GAC*SERSMNF*CG (peptide sequence of this compound: SEQ ID NO. 103)

Sequences given in italics are targeting sequences, i.e. the airway epithelium ligand *GACSERSMNFCG* or *GACYGLPHKFCG,* the nuclear localization sequence (NLS) *VKRKKKP,* the high-density lipoprotein (HDL)-mimetic peptide *FAEKFKEAVKDYFAKFWD*GSG as scavenger receptor B1 ligand, the dendritic cell ligand *FYPSYHSTPQRP,* D-Mannose, *CYTYQGKLC* or *TITHFQFGPTVY*, the flagellin-related peptide *INNNLQRVRELAVQSANSTNSQSDLDS,* the microfold cell ligand *CKSTHPLSC, CRGDKGPDC, KANSSYSGNYPYSILFQKF, KIAIVNVSSIFQQLPARET* or *FHQLPARSPLP,* or the LTB-related peptide *LDAGQYVLVM.* Underlined sequences represent hydrophobic moieties. Sequences given in bold represent protein or polypeptide binding moieties.

In compounds P6, P8, P11 and P12 the hydrophobic moiety in form of the lipoic acid residue, the C₁₃H₂₇CONH-residue and the cholesteryl is in each case covalently bound to the N-terminus of the peptide residues according to SEQ ID NO. 54 and SEQ ID NO. 53.

Binary complexes comprising the synthetic compound and the protein or the polypeptide, and ternary complexes comprising the synthetic compound, the protein or the polypeptide and the amphiphilic block copolymer, were characterized. The binary complexes and ternary complexes were characterized physically, in particular by measuring the arithmetic average particle diameter and the polydispersity index (PDI) using a particle size meter. In order to investigate the effect of concentration of the synthetic compound on the binary complex or on the ternary complex, equal volumes of the synthetic compound at different concentrations from 0.15 µg/µL to 5 µg/µL were used for preparation of the binary complex and the ternary complex. In order to investigate the effect of concentration of the protein or the polypeptide on the binary complex or on the ternary complex, equal volumes of the protein or the polypeptide at different concentrations from 0.25 µg/µL to 0.67 µg/µL were used for preparation of the binary complex and the ternary complex. In order to investigate the effect of concentration of the amphiphilic block copolymer on the binary complex or on the ternary complex, equal volumes of the amphiphilic block copolymer or the amphiphilic lipopeptide at different concentrations from 0.5 µg/µL to 5 µg/µL were used for preparation of the ternary complex.

Furthermore, in order to investigate the effect of the solution conditions on the arithmetic average particle diameter of the binary complexes and the ternary complexes, the complexes were prepared in either double-distilled water, 0.5 mM HEPES buffer solution, 1 mM HEPES buffer solution or 10 mM HEPES buffer solution.

In the following, binary complexes containing compound 1 or compound 2 as synthetic compound and ovalbumin (OVA) as protein are exemplarily shown. Furthermore, ternary complexes containing compound 1 or compound 2 as synthetic compound, ovalbumin (OVA) as protein and the amphiphilic block copolymers T904, T704, T90R4 or T304 are exemplarily shown. The inventors found that binary complexes containing other synthetic compounds and/or other proteins or polypeptides showed similar characteristics as binary complexes containing compounds 1 or 2 and OVA. The inventors also found that ternary complexes containing other synthetic compounds and/or other proteins or polypeptides and/or amphiphilic lipopeptides showed similar characteristics as ternary complexes containing compounds 1 or 2, OVA and an amphiphilic block copolymer.

The results are given in the following tables:

**Table 1: Complex preparation in double-distilled water (H₂O)**

| | **Average arithmetic particle diameter** | **PDI** |
|---|---|---|
| **Binary complex** | | |
| Compound 2 (1.6 µg/µL) + OVA (0.3 µg/µL) | 895.3 nm | 0.225 |
| Compound 2 (1.5 µg/µL) + OVA (0.5 µg/µL) | 1875 nm | 0.22 |
| Compound 2 (1.33 µg/µL) + OVA (0.67 µg/µL) | 2622 nm | 0.142 |
| | | |

| **Ternary complex** | | |
|---|---|---|
| Compound 1 (5 µg/µL) + OVA (0.4 µg/µL) + T904 (2.5 µg/µL) | 1800 nm | 0.36 |
| Compound 1 (5 µg/µL) + OVA (1.0 µg/µL) + T904 (5 µg/µL) | 2400 nm | 0.16 |
| Compound 1 (2.5 µg/µL) + OVA (2.5 µg/µL) + T904 (2.5 µg/µL) | 2300 nm | 0.2 |

**Table 2: Complex preparation in 0.5 mM HEPES buffer solution**

| | **Arithmetic average particle diameter** | **PDI** |
|---|---|---|
| **Binary complex** | | |
| Compound 1 (0.9 µg/µL) + OVA (0.3 µg/µL) | 300 nm | 0.628 |
| Compound 1 (0.6 µg/µL) + OVA (0.3 µg/µL) | 880 nm | 0.47 |
| Compound 1 (0.3 µg/µL) + OVA (0.3 µg/µL) | 2357 nm | 0.33 |
| Compound 1 (0.15 µg/µL) + OVA (0.3 µg/µL) | 401 nm | 0.189 |

| **Ternary complex** | | |
|---|---|---|
| Compound 2 (0.15 µg/µL) + OVA (0.15 µg/µL) + T704 (0.5 µg/µL) | 110 nm | 0.147 |
| Compound 2 (0.15 µg/µL) + OVA (0.15 µg/µL) + T704 (1.0 µg/µL) | 104 nm | 0.221 |
| Compound 2 (0.15 µg/µL) + OVA (0.15 µg/µL) + T904 (0.625 µg/µL) | 105 nm | 0.167 |

**Table 3: Complex preparation in 1 mM HEPES buffer solution**

| | **Arithmetic average particle diameter** | **PDI** |
|---|---|---|
| **Binary complex** | | |
| Compound 1 (1.6 µg/µL) + OVA (0.25 µg/µL) | 227 nm | 0.65 |
| Compound 1 (1.6 µg/µL) + OVA (0.4 µg/µL) | 327 nm | 0.628 |
| Compound 1 (1.5 µg/µL) + OVA (0.5 µg/µL) | 272.3 nm | 0.551 |
| Compound 1 (1.33 µg/µL) + OVA (0.67 µg/µL) | 405 nm | 0.377 |
| Compound 2 (0.15 µg/µL) + OVA (0.3 µg/µL) | 99.11 nm | 0.24 |

**Table 4: Complex preparation in 10 mM HEPES buffer solution**

| | **Arithmetic average particle diameter** | **PDI** |
|---|---|---|
| **Binary complex** | | |
| Compound 1 (0.15 µg/µL) + OVA (0.3 µg/µL) | 279 nm | 0.124 |
| Compound 2 (0.15 µg/µL) + OVA (0.3 µg/µL) | 94.29 nm | 0.347 |

| **Ternary complex** | | |
|---|---|---|
| Compound 2 (0.15 µg/µL) + OVA (0.3 µg/µL) + T90R4 (0.5 µg/µL) | 72 nm | 0.59 |
| Compound 2 (0.2 µg/µL) + OVA (0.2 µg/µL) + T90R4 (0.5 µg/µL) | 330 nm | 0.253 |
| Compound 2 (0.3 µg/µL) + OVA (0.2 µg/µL) + T90R4 (0.5 µg/µL) | 280 nm | 0.131 |
| Compound 2 (0.3 µg/µL) + OVA (0.3 µg/µL) + T90R4 (0.5 µg/µL) | 115.8 nm | 0.251 |
| Compound 2 (0.3 µg/µL) + OVA (0.3 µg/µL) + T704 (0.5 µg/µL) | 129 nm | 0.284 |
| Compound 2 (0.6 µg/µL) + OVA (0.3 µg/µL) + T704 (1.0 µg/µL) | 259 nm | 0.054 |
| Compound 2 (0.9 µg/µL) + OVA (0.3 µg/µL) + T704 (0.5 µg/µL) | 332 nm | 0.017 |
| Compound 2 (0.15 µg/µL) + OVA (0.3 µg/µL) + T304 (0.5 µg/µL) | 200 nm | 0.486 |
| Compound 2 (0.3 µg/µL) + OVA (0.3 µg/µL) + T304 (0.5 µg/µL) | 122 nm | 0.342 |
| Compound 2 (0.2 µg/µL) + OVA (0.2 µg/µL) + T904 (0.5 µg/µL) | 236 nm | 0.202 |
| Compound 2 (0.3 µg/µL) + OVA (0.2 µg/µL) + T904 (0.5 µg/µL) | 234 nm | 0.084 |

As can be seen in Tables 1 to 4, binary complexes and ternary complexes prepared in double-distilled water show a relatively large arithmetic average particle diameter compared to binary complexes and ternary complexes prepared in HEPES buffer solutions. Furthermore, with respect to binary complex preparation, a relatively high concentration of HEPES in the HEPES buffer solution, in particular 1 mM and 10 mM HEPES buffer solution, results in relatively small arithmetic average particle diameter of the binary complex. Furthermore, with respect to ternary complex preparation, both a 0.5 mM HEPES buffer solution and a 10 mM HEPES buffer solution results in relatively small arithmetic average particle diameters of the ternary complex. Ternary complex particles with a relatively small arithmetic average particle diameter, in particular an arithmetic average particle diameter of at most 500 nm, in particular of at most 400 nm, in particular of at most 300 nm, in particular of at most 200 nm, in particular of at most 100 nm, show a good distribution and cellular uptake profile. Furthermore, ternary complex particles with a relatively small arithmetic average particle diameter show a relatively low trapping and degradation, for example by the mucus layer, compared to ternary complex particles with a relatively large arithmetic average particle diameter. Thus, ternary complex particles with a relatively small arithmetic average particle diameter can for example efficiently travel through the mucus layer and can be efficiently internalized by respiratory epithelium cells and/or immune cells.

As can be seen in Table 2, ternary complex preparation in 0.5 mM HEPES buffer solution containing the amphiphilic block copolymer T704 in two different concentrations or the amphiphilic block copolymer T904 resulted in a similar relatively small arithmetic average particle diameter of the ternary complex.

To show the effect of the solvent type on the arithmetic average particle diameter of the ternary complex, ternary complexes containing ovalbumin, poloxamine 704 and compound 2 were prepared via a two-step incubation process described as follows:
Briefly, the solution of the amphiphilic block copolymer or the poloxamine 704 at a concentration of 4 µg/µLin a specific solvent (e. g. PBS, double distilled water, 0.05 M Tris or 5 mM HEPES) was mixed with a dilution of the ovalbumin at a specific concentration (e. g. 1.2 µg/µL) with a v/v = 2:1. After 20 min incubation at 4 °C, a dilution of compound 2 at a concentration of 1.2 µg/µL in the same solvent was added to the above mixture with a v/v = 1:3 and mixed gently by pipetting up and down several times. Subsequently, the ternary complex was incubated for 20 minutes at 4 °C before further use.

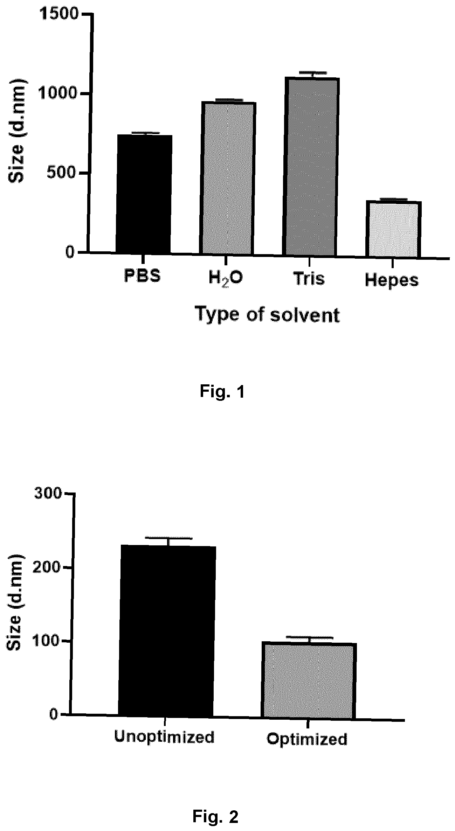

The ternary complexes were either prepared in PBS, in double distilled water, in 0.05M Tris buffer or in 5 mM HEPES buffer. Subsequently, ternary complex arithmetic average particle diameters were determined. The results are shown in Fig. 1.

As can be seen in Fig. 1, the arithmetic average particle diameter of the ternary complex is dependent on the solvent used during preparation of the ternary complex. Ternary complexes prepared in PBS resulted in an arithmetic average particle diameter of 734 nm. Ternary complexes prepared in double-distilled water (H₂O) and in 0.05 M Tris buffer (Tris) resulted in arithmetic average particle diameters of 944 nm and 1110 nm, respectively. In contrast to ternary complex preparation in PBS, H₂O or Tris, ternary complex preparation in 5 mM HEPES buffer solution (Hepes) resulted in arithmetic average particle diameters of 368 nm. These results indicate that ternary complex preparation in a HEPES buffer solution results in a relatively small arithmetic average particle diameter of the ternary complex. These results further indicate that the preparation of the ternary complex in a buffer system in general does not automatically result in a relatively small arithmetic average particle diameter of the ternary complex. This is particularly shown for ternary complexes prepared in 0.05 M Tris buffer which resulted in a relatively big arithmetic average particle diameter compared to ternary complexes prepared in double-distilled water.

For showing the effect of the preparation method on the arithmetic average particle diameter of the ternary complex, ternary complexes containing OVA, poloxamine T704 and compound 2 were prepared according to the following methods:
Method 1: Mixing of OVA (0.3 µg/µL) and poloxamine T704 (0.5 µg/µL) and subsequent addition of compound 2 (0.15 µg/µL). The particles were prepared in 0.5 mM HEPES buffer solution. The particles were characterized immediately.
   or
Method 2: Mixing of compound 2 (0.15 µg/µL) and poloxamine T704 (0.5 µg/µL). Addition of OVA (0.3 µg/µL) after 30 minutes. The particles were prepared in 10 mM HEPES buffer solution. The particles were characterized after 3 hours incubation.

Ternary complex preparation according to method 1 resulted in a ternary complex arithmetic average particle diameter of 162 nm and a PDI of 0.169. Ternary complex preparation according to method 2 resulted in a ternary complex arithmetic average particle diameter of 87 nm and a PDI of 0.374. This indicates that method 2 results in a relatively small arithmetic average particle diameter of the ternary complex.

In another experiment, the following methods were compared with respect to ternary complex preparation:
Method 3: Mixing of compound 2 (0.3 µg/µL) and OVA (0.3 µg/µL) and subsequent addition of poloxamine T704 (1 µg/µL). The particles were prepared in 0.5 mM HEPES buffer solution. The particles were characterized immediately.
   or
Method 4: Mixing of OVA (0.3 µg/µL) and poloxamine T704 (1 µg/µL) and subsequent addition of compound 2 (0.3 µg/µL). The particles were prepared in 0.5 mM HEPES buffer solution. The particles were characterized immediately.

The results are shown in Fig. 2. As can be seen in Fig. 2, ternary complex preparation according to method 3 (unoptimized) resulted in an arithmetic average particle diameter of the ternary complex of 231 nm. Ternary complex preparation according to method 4 (optimized) resulted in an arithmetic average particle diameter of the ternary complex of 101 nm. These results indicate that mixing of the protein with the amphiphilic block copolymer and subsequent addition of the synthetic compound results in a relatively small arithmetic average particle diameter of the ternary complex.

### In vitro Experiments:

16HBE (human bronchial epithelial cell line) cells were cultured in Eagle's Minimal Essential Medium (Gibco) with 10 % heat-inactivated FBS (Gibco) and 1 % (v/v) penicillin/streptomycin (Gibco) at 37 °C and 5 % CO₂. In order to study the cellular uptake of ternary complexes, 16HBE cells (7.5 × 10⁵/well) were seeded in a 6-well plate 24 h before investigation. 16HBE cells were incubated with FITC-conjugated ovalbumin (FITC-OVA, OVA1-FC-1, NANOCS) or ternary complex FITC-OVA/T90R4/C2 for 4 h in Opti-MEM Medium (31985062, Invitrogen). The amount of FITC-OVA in each sample was 4 µg/wel. PBS-treated sample served as a negative control. The cells were harvested and rinsed with cold PBS for three times followed immediately by flow cytometry analysis. The amount of 10.000 live cells were collected for each sample.

### FITC-conjugated ovalbumin (Fluo-OVA):

30 µg FITC-OVA formulated in 0.5 mM HEPES solution (pH 6.35).

### FITC-OVA/T90R4/C2 (Ternary complex Fluo-OVA/T90R4/C2):

30 µg OVA formulated with 250 µg T90R4 and 30 µg compound 2 in 0.5 mM HEPES solution (pH 6.35).

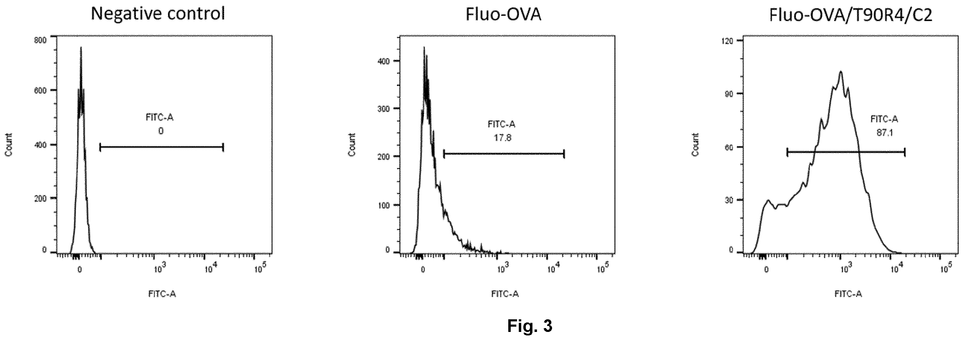

The FITC fluorescence intensity within the cells after incubation with Fluo-OVA and Fluo-OVA/T90R4/C2 ternary complex are shown in Fig. 3. PBS incubated samples are shown as negative control (Fig. 3, left panel).

As shown in the middle panel of Fig. 3, the fluorescein-conjugated ovalbumin (Fluo-OVA) was internalized by 17.8 % of 16HBE cells after 4 h incubation. In contrast, the Fluo-OVA/T90R4/C2-based ternary complex significantly improved the cellular uptake of FITC-conjugated OVA showing internalization by 87.1 % of 16HBE cells (Fig. 3, right panel). Thus, incubation of 16HBE cells with the Fluo-OVA/T90R4/C2 based ternary complex results in an improved cellular uptake of fluorescein-conjugated ovalbumin.

### In vivo Experiments:

The following vaccination experiment was performed: BALB/c mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were instilled intratracheally as a bolus (50 µL/mouse) through a 22-gauge catheter (priming vaccination, day 0):

### P407(13 %)-C2-CpG (Ternary complex + immunologic adjuvant):

15 µg OVA formulated with 13 % P407, 15 µg compound 2 and 5 µg CpG. CpG is an immunologic adjuvant comprising cytosine-phospho-guanine (CpG) motifs. CpG is a synthetic form of DNA that mimics bacterial and viral genetic material. If included in a vaccine, CpG improves an immune response directed against an antigen.

### MF59-OVA (immunologic adjuvant):

15 µg OVA formulated with 30 µg MF59. MF59 is an oil-in-water emulsion and an immunologic adjuvant. One dose of MF59 contains 9.75 mg squalene, 1.175 mg surface-active polysorbate 80 (Tween 80), 1.175 mg sorbitan trioleate (Span 85), sodium citrate, and citric acid. If included in a vaccine, MF59 improves an immune response directed against an antigen. The ternary complex was prepared in 0.5 mM HEPES at pH 6.35.

### T90R4-C2-CpG (Ternary complex + immunologic adjuvant):

15 µg OVA formulated with 125 µg T90R4, 15 µg compound 2 and 5 µg CpG. The ternary complex was prepared in 0.5 mM HEPES at pH 6.35.

### P407(10 %)-C2 (Ternary complex):

15 µg OVA formulated with 10 % P407 and 15 µg compound 2. The ternary complex was prepared in 0.5 mM HEPES at pH 6.35.

### T90R4-C2 (Ternary complex):

15 µg OVA formulated with 125 µg T90R4 and 15 µg compound 2. The ternary complex was prepared in 0.5 mM HEPES at pH 6.35.

### T904-C2 (Ternary complex):

15 µg OVA formulated with 125 µg T904 and 15 µg compound 2. The ternary complex was prepared in 0.5 mM HEPES at pH 6.35.

Mice receiving PBS (50 µL/mouse) were studied as negative controls. Each group contained five BALB/c mice (n=5).

Mice received two booster immunizations by intratracheal administration of formulations identical to the priming formulation on day 14 after priming and on day 28 after priming, respectively. On day 35, mice were sacrificed and serum samples and vaginal lavage fluid samples collected. OVA-specific IgG antibodies were determined in serum samples (day 35). OVA-specific IgA antibodies were determined in vaginal lavage fluid samples (day 35).

In order to detect OVA-specific IgG and IgA antibodies, ELISA plates (Corning) were coated overnight with 8 mg/L ovalbumin in 0.05 M sodium carbonate-sodium bicarbonate buffer, pH 9.6, and blocked in 5 % skimmed milk in PBS. Diluted serum (dilution 1:1000) or vaginal lavage fluid samples (dilution 1:4) were added to each well. Plates were incubated with goat anti-mouse IgG-HRP or IgA-HRP antibodies and developed with 3,3',5,5'-tetramethylbenzidine (TMB) substrate. Reactions were stopped with 2 M hydrochloric acid, and the absorbance was measured at 450 nm using a microplate reader (PerkinElmer, USA). The endpoint titers were defined as the highest reciprocal dilution of serum to yield an absorbance greater than 2.1-fold of the background values.

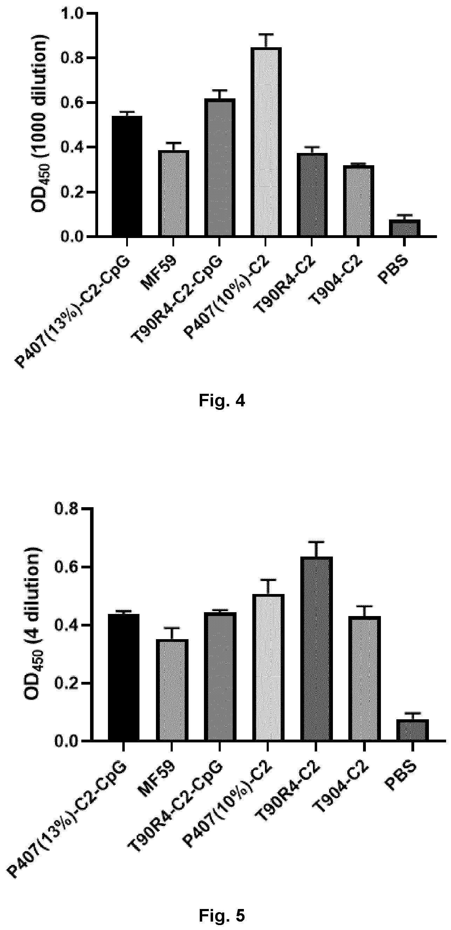

Results are shown in Fig. 4 for OVA-specific IgG antibodies in serum and in Fig. 5 for OVA-specific IgA antibodies in vaginal lavage fluid.

As can be seen in Fig. 4, all the formulations containing OVA induced the production of OVA-specific IgG antibodies within serum samples. The T90R4-C2-CpG group and P407(10 %)-C2 group displayed much higher serum IgG levels compared to MF59 group.

As can be seen in Fig. 5, all the formulations containing OVA induced the production of OVA-specific IgA antibodies within vaginal lavage fluid samples. The T90R4-C2 group showed the highest IgA level within vaginal lavage fluid samples compared to the other groups.

In another vaccination experiment, BALB/c mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were instilled intratracheally as a bolus (20 µL/mouse) through a 22-gauge catheter (priming vaccination, day 0):

### RBD/P407/C2 (Ternary complex):

6 µg of the receptor binding domain within the spike protein of SARS-CoV-2 virus (RBD) formulated with 13 % P407 and 6 µg compound 2. The ternary complex was prepared in 0.5 mM HEPES at pH 6.35.

### L-RBD/P407/C2 (Ternary complex + immunologic adjuvant):

Heat-labile enterotoxin (LTB) was linked to RBD via the linker GSGSG to form the fusion protein LTB-RBD (L-RBD). LTB in LTB-RBD is an intramolecular immunologic adjuvant improving an immune response directed against RBD. The formulation contained 6 µg of L-RBD formulated with 13 % P407 and 6 µg compound 2. The ternary complex was prepared in 0.5 mM HEPES at pH 6.35.

### RBD/T90R4/C2 (Ternary complex):

6 µg of RBD formulated with 50 µg T90R4 and 6 µg compound 2. The ternary complex was prepared in 0.5 mM HEPES at pH 6.35.

### L-RBD/T90R4/C2 (Ternary complex + immunologic adjuvant):

6 µg of L-RBD formulated with 50 µg T90R4 and 6 µg compound 2. The ternary complex was prepared in 0.5 mM HEPES at pH 6.35.

### RBD:

6 µg of RBD in 0.5 mM HEPES at pH 6.35.

### LTB-RBD:

6 µg of L-RBD in 0.5 mM HEPES at pH 6.35.

Mice receiving PBS (20 µL/mouse) were studied as negative controls. Each group contained five BALB/c mice (n=5).

Mice received two booster immunizations by intratracheal administration of formulations identical to the priming formulation on day 14 after priming and on day 28 after priming, respectively. On days 14, 21, 28, 35 and 42, serum samples and vaginal lavage fluid samples were collected. On day 52, mice were sacrificed and serum samples and bronchoalveolar lavage fluid (BALF) samples were collected. RBD-specific IgG antibodies were determined in serum samples (days 14, 21, 28, 35 and 42). RBD-specific IgA antibodies were determined in vaginal lavage fluid samples (days 14, 21, 28, 35 and 42) and BALF samples (day 52). Pseudovirus neutralization and anti-SARS-CoV-2 neutralizing antibody titers were determined in serum samples (day 52) and BALF samples (day 52).

In order to detect RBD-specific IgG and IgA antibodies, ELISA plates (Corning) were coated overnight with 2 µg/mL of SARS-CoV-2 RBD recombinant protein in 0.05 M carbonate-bicarbonate buffer, pH 9.6, and blocked in 5 % skimmed milk in PBS. Diluted serum samples (dilution 1:1000), vaginal lavage fluid samples (dilution 1:4) or BALF samples (dilution 1:4) were added to each well. Plates were incubated with goat anti-mouse IgG-HRP or IgA-HRP antibodies and developed with 3,3',5,5'-tetramethylbenzidine (TMB) substrate. Reactions were stopped with 2 M hydrochloric acid, and the absorbance was measured at 450 nm using a microplate reader (PerkinElmer, USA). The endpoint titers were defined as the highest logarithmic dilution of serum to yield an absorbance greater than 2.1-fold of the background values.

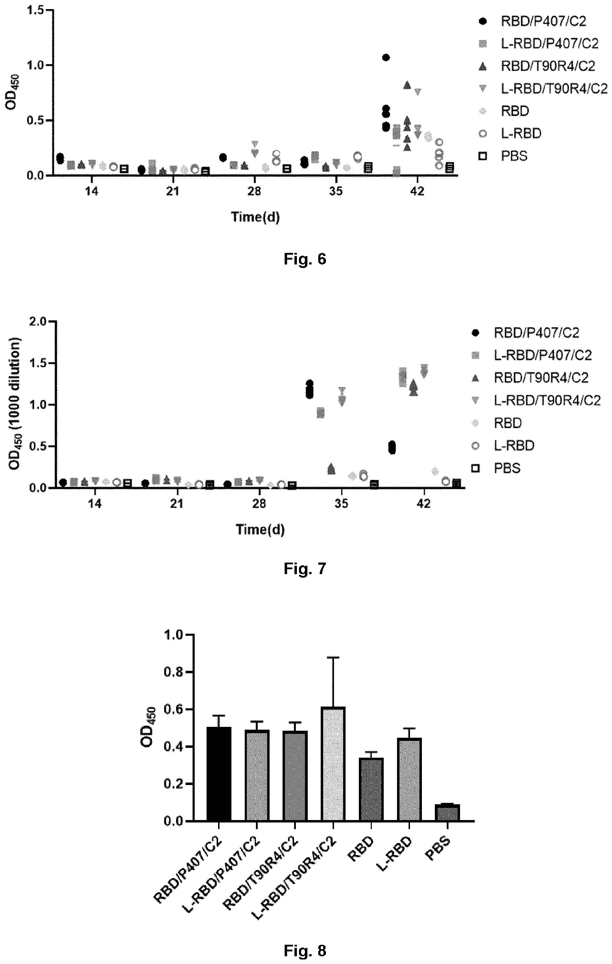

Results are shown in Fig. 6 for RBD-specific IgG antibodies in serum samples (day 14, day 21, day 28, day 35 and day 42), in Fig. 7 for RBD-specific IgA antibodies in vaginal lavage fluid samples (day 14, day 21, day 28, day 35 and day 42) and in Fig. 8 for RBD-specific IgA antibodies in BALF samples (day 52).

As can be seen in Fig. 6, all the formulations containing RBD or L-RBD induced the production of RBD-specific IgG antibodies within serum samples from day 35. The RBD/P407/C2 group, the L-RBD/P407/C2 group and the L-RBD/T90R4/C2 group show the highest induction of RBD-specific IgG antibodies. Although the serum IgG antibody level of the RBD/P407/C2 group decreased substantially from day 35 to day 42, the L-RBD/P407/C2 group, the RBD/T90R4/C2 group and the L-RBD/T90R4/C2 group showed high levels of RBD-specific IgG antibodies within serum samples on day 42. Notably, both the L-RBD/P407/C2 group and the L-RBD/T90R4/C2 group showed a high level induction of RBD-specific IgG antibodies within serum samples on day 35 and day 42.

As can be seen in Fig. 7, on day 42 the RBD-specific IgA antibodies within vaginal lavage fluid samples significantly increased in mice immunized with the RBD/P407/C2 based ternary complex, the RBD/T90R4/C2 based ternary complex and the L-RBD/T90R4/C2 based ternary complex.

As can be seen in Fig. 8, all the formulations containing RBD or L-RBD efficiently induced RBD-specific IgA antibodies within bronchoalveolar lavage fluid (BALF) samples. The samples from the L-RBD/T90R4/C2 group showed the highest IgA level within BALF samples compared to all the other groups.

In order to measure pseudovirus neutralization, mouse serum and BALF samples from mice intranasally immunized with the L-RBD/T90R4/C2 based ternary complex were serially diluted using high-glucose DMEM buffer containing 2 % fetal bovine serum (FBS). HEK293T cells stably expressing ACE2 (hACE2-293T) were seeded into 384-well plates (5×10³ per well) and grown overnight. The diluted serum samples or BALF samples were mixed with 100 TCIDso of EGFP-SARS-CoV-2 pseudotyped virus (BPS-79981-2, Biomol, Germany) and incubated for 60 min at 37 °C. The mixtures were transferred to the 384-well plates seeded with the HEK293T cells. After incubation for 24 hours, 2 % FBS high-glucose DMEM was replaced with 10 % FBS high-glucose DMEM to allow sufficient GFP expression for the next 48 hours. Finally, high content analysis was performed on Opera Phenix (PerkinElmer). Each well was imaged and calculated with an average fluorescence intensity of 488 nm (AFI488).

The inhibition percentage of each well was calculated with the formula inhibition (%) = (1-(AFI488_{assay}-AFI488_{blank control})/(AFI488_{infection control}-AFI488_{blank control}))×100%. The 50 % inhibition titer (IT₅₀) was calculated with a dose-response inhibition (normalized response) model by GraphPad Prism 8.0.

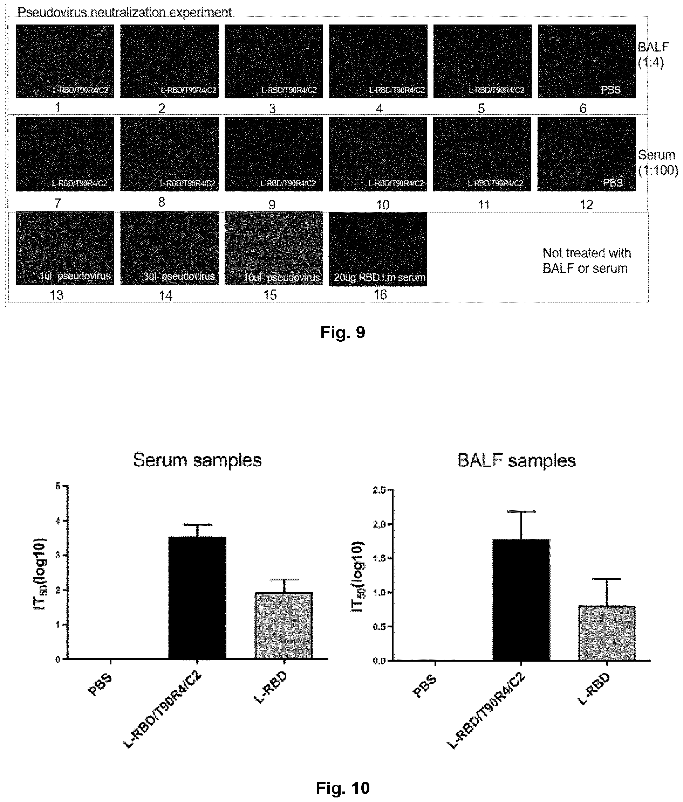

The results are shown in Fig. 9 and Fig. 10. Fig. 9 shows typical images of the pseudovirus neutralization assay. The images 1 to 6 refer to the pseudovirus neutralization of the BALF samples (1:4 dilution) of mice immunized either with the L-RBD/T90R4/C2 (L-RBD: 0.3 µg/µL; T90R4: 2.5 µg/µL: C2: 0.3 µg/µL) based ternary complex (images 1 to 5) or treated with PBS (image 6). The images 7 to 12 refer to the pseudovirus neutralization of the serum samples (1:100 dilution) of mice immunized either with the L-RBD/T90R4/C2 (L-RBD: 0.3 µg/µL; T90R4: 2.5 µg/µL; C2: 0.3 µg/µL) based ternary complex (images 7 to 11) or with PBS (image 12). The images 13 to 15 refer to pseudovirus infection groups which were not incubated with BALF samples or serum samples. Image 16 refers to the pseudovirus neutralization of the serum sample (1:100 dilution) of a mouse immunized with 20 µg RBD together with alum adjuvant via intramuscular injection. Fig. 10 shows the SARS-CoV-2 pseudovirus neutralizing antibody titers in serum samples (left panel) and BALF samples (right panel) on day 52 (n=3).

As can be seen in Fig. 9, the wells without BALF sample or serum sample incubation (images 13 to 15) show HEK293T cells infected with the SARS-CoV-2 pseudovirus. Also the wells incubated with the serum sample (image 6) or the BALF sample (image 12) of a mouse treated with PBS show HEK293T cells infected with the SARS-CoV-2 pseudovirus. The wells incubated with BALF samples (images 1 to 5) or serum samples (images 7 to 11) of mice immunized with the L-RBD/T90R4/C2 based ternary complex show either a reduced infection of HEK293T cells or no infection. In this regard, the serum samples were able to block the infection of HEK293T cells more efficiently than the BALF samples. Furthermore, the well incubated with the serum sample of a mouse immunized with 20 µg RBD together with alum adjuvant shows a reduced infection.

As can be seen in Fig. 10, the serum samples and BALF samples from mice treated with PBS could not inhibit pseudovirus infection. Immunization with the L-RBD/T90R4/C2-based ternary complex results in an increased IT₅₀ of anti-SARS-CoV-2 neutralizing antibodies in both serum and BALF samples compared to mice immunized with L-RBD only. These results indicate that immunization with the ternary complex results in a neutralizing humoral immune response.

In another vaccination experiment, BALB/c mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were instilled intratracheally as a bolus through a 22-gauge catheter (priming vaccination, day 0):

### L-RBD/T90R4/C1 18ug i.t (Ternary complex):

18 µg of L-RBD formulated with 150 µg T90R4 and 18 µg compound 1. The ternary complex was prepared in 0.5 mM HEPES at pH 6.35. Per mouse, 60 µL of the formulation were administered.

### RBD/T90R4/C2 18ug i.t (Ternary complex):

18 µg of RBD formulated with 150 µg T90R4 and 18 µg compound 2. The ternary complex was prepared in 0.5 mM HEPES at pH 6.35. The formulation was administered in a volume of 60 µL.

The following formulations were administered as intratracheal spray using a highpressure MicroSprayer device (Penn Century Inc., Wyndmoor USA):

### L-RBD/T90R4/C2 18ug i.t (Ternary complex):

18 µg of L-RBD formulated with 150 µg T90R4 and 18 µg compound 2. The ternary complex was prepared in 0.5 mM HEPES at pH 6.35. The formulation was administered in a volume of 60 µL.

### L-RBD/T90R4/C2 12ug i.t (Ternary complex):

12 µg of L-RBD formulated with 100 µg T90R4 and 12 µg compound 2. The ternary complex was prepared in 0.5 mM HEPES at pH 6.35. The formulation was administered in a volume of 40 µL.

### L-RBD/T90R4/C2 9ug i.t (Ternary complex):

9 µg of L-RBD formulated with 75 µg T90R4 and 9 µg compound 2. The ternary complex was prepared in 0.5 mM HEPES at pH 6.35. The formulation was administered in a volume of 30 µL.

The following formulation was injected intramuscularly via the administration to the tibialis anterior muscle of mice by needle injection:

### L-RBD/T90R4/C2 18ug i.m (Ternary complex):

18 µg of L-RBD formulated with 150 µg T90R4 and 18 µg compound 2. The ternary complex was prepared in 0.5 mM HEPES at pH 6.35.The formulation was administered in a volume of 60 µL.

The following formulations was instilled intranasally into the nostrils of mice as a bolus through a 100 µL pipette with a tip:

### L-RBD/T90R4/C2 18ug i.n (Ternary complex):

18 µg of L-RBD formulated with 150 µg T90R4 and 18 µg compound 2. The ternary complex was prepared in 0.5 mM HEPES at pH 6.35. The formulation was administered in a volume of 60 µL.

Mice receiving PBS intratracheally (60 µL/mouse) were studied as negative controls. Each group contained five BALB/c mice (n=5).

Mice received two booster immunizations by the same route of administration as in the priming immunization with formulations identical to the priming formulation on day 14 after priming and on day 28 after priming, respectively. On days 35 and 45, serum samples were collected. On day 35, vaginal lavage fluid samples were collected. On day 45, mice were sacrificed and pulmonary tissue samples were collected. RBD-specific IgG antibodies were determined in serum samples (days 35 and 45). RBD-specific IgA antibodies were determined in vaginal lavage fluid samples (day 35) and pulmonary tissue samples (day 45).

Sample collection was performed as follows: Blood samples were collected on specific time points as indicated above. The bronchoalveolar lavage fluids were collected by washing the lungs of euthanized mice with a mucosal preparation solution (MPS) consisting of 700-1000 µL of ice-cold PBS containing 100 µM EDTA, 0.05 % sodium azide, 0.05 % Tween-20, and 1× protease inhibitor (Pierce^{™} Protease Inhibitor Tablets) using a blunt-ended needle. The vaginal lavage fluids were obtained by rinsing mice four times with each 75 µl of MPS. All the samples collected were centrifuged at 8000 × g for 10 min at 4 °C and the supernatants were stored at -80 °C until the ELISA assay is performed.

The RBD-specific ELISA was performed as follows: ELISA plates (Corning) were coated overnight with 2 µg/mL of SARS-CoV-2 RBD recombinant protein in 0.05 M carbonate-bicarbonate buffer, pH 9.6, and blocked with 5 % skimmed milk in PBS. Diluted serum samples or lavage fluid samples were added to each well. Plates were incubated with goat anti-mouse IgG-HRP or IgA-HRP antibodies and developed with 3,3',5,5'-tetramethylbenzidine (TMB) substrate. Reactions were stopped using 2 M hydrochloric acid, and the absorbance was measured at 450 nm using a microplate reader (PerkinElmer, USA). The endpoint titers were defined as the highest logarithmic dilution of serum or lavage fluids samples to yield an absorbance greater than 2.1-fold of the background values.

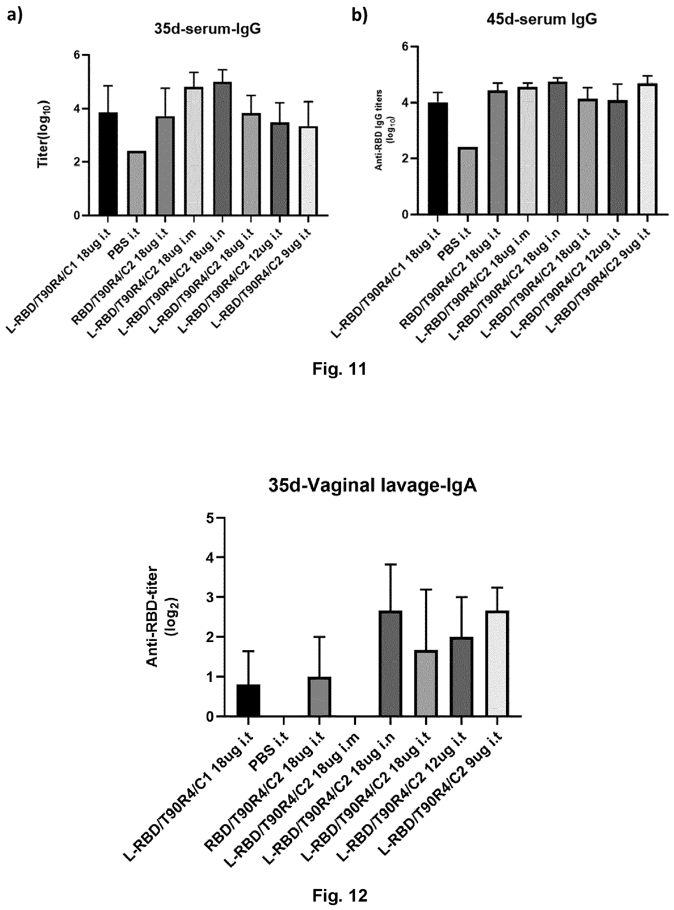

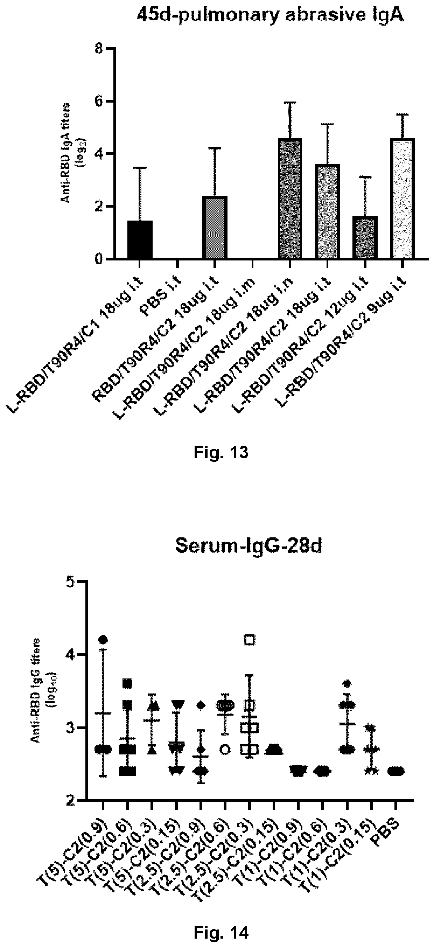

Results are shown in Figs. 11a and 11b for RBD-specific IgG antibodies in serum samples (day 35 and day 45), in Fig. 12 for RBD-specific IgA antibodies in vaginal lavage fluid samples (day 35) and in Fig. 13 for RBD-specific IgA antibodies in pulmonary tissue samples (day 45).

As can be seen in Fig. 11a, all the formulations containing RBD or L-RBD induced the production of RBD-specific IgG antibodies within serum samples. The intramuscularly injected L-RBD/T90R4/C2-based ternary complex (L-RBD/T90R4/C2 18 ug i.m.) and intranasally inoculated L-RBD/T90R4/C2-based ternary complex (L-RBD/T90R4/C2 18 ug i.n.) resulted in the highest production of RBD-specific IgG antibodies on day 35. Furthermore, as can be seen in Fig. 11b, all the ternary complex formulations containing RBD or L-RBD induced comparable levels of RBD-specific IgG antibodies within serum samples on day 45.

As can be seen in Fig. 12, only intranasally or intratracheally inoculated ternary complex formulations containing RBD or L-RBD resulted in the production of RBD-specific IgA antibodies within vaginal lavage fluid samples. The RBD-specific IgA antibody levels within samples from the intranasally inoculated L-RBD/T90R4/C2-based ternary complex formulation (L-RBD/T90R4/C2 18 ug i.n.) and the intratracheally administered L-RBD/T90R4/C2-based ternary complex formulation (L-RBD/T90R4/C2 9 ug i.t.) show the highest production of RBD-specific IgA antibodies within vaginal lavage fluid samples.

As can be seen in Fig. 13, only intranasally or intratracheally inoculated ternary complex formulations containing RBD or L-RBD resulted in the production of RBD-specific IgA antibodies in the lungs of immunized mice. The intranasally inoculated L-RBD/T90R4/C2-based ternary complex formulation (L-RBD/T90R4/C2 18 ug i.n.) and the intratracheally administered L-RBD/T90R4/C2-based ternary complex formulation (L-RBD/T90R4/C2 9 ug i.t.) show the highest production of RBD-specific induce the highest levels of RBD-specific IgA antibodies within the lungs of immunized mice.

In a further vaccination experiment, BALB/c mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were administered intranasally (40 µL/mouse) as a bolus through a 100 µL pipette with a tip (priming vaccination, day 0):
T(5)-C2(0.9):
   12 µg of L-RBD formulated with 200 µg T90R4 and 36 µg compound 2. The ternary complex was prepared in 0.5 mM HEPES at pH 6.35.
T(5)-C2(0.6):
   12 µg of L-RBD formulated with 200 µg T90R4 and 24 µg compound 2. The ternary complex was prepared in 0.5 mM HEPES at pH 6.35.
T(5)-C2(0.3):
   12 µg of L-RBD formulated with 200 µg T90R4 and 12 µg compound 2. The ternary complex was prepared in 0.5 mM HEPES at pH 6.35.
T(5)-C2(0.15):
   12 µg of L-RBD formulated with 200 µg T90R4 and 6 µg compound 2. The ternary complex was prepared in 0.5 mM HEPES at pH 6.35.
T(2.5)-C2(0.9):
   12 µg of L-RBD formulated with 100 µg T90R4 and 36 µg compound 2. The ternary complex was prepared in 0.5 mM HEPES at pH 6.35.
T(2.5)-C2(0.6):
   12 µg of L-RBD formulated with 100 µg T90R4 and 24 µg compound 2. The ternary complex was prepared in 0.5 mM HEPES at pH 6.35.
T(2.5)-C2(0.3):
   12 µg of L-RBD formulated with 100 µg T90R4 and 12 µg compound 2. The ternary complex was prepared in 0.5 mM HEPES at pH 6.35.
T(2.5)-C2(0.15):
   12 µg of L-RBD formulated with 100 µg T90R4 and 6 µg compound 2. The ternary complex was prepared in 0.5 mM HEPES at pH 6.35.
T(1)-C2(0.9):
   12 µg of L-RBD formulated with 40 µg T90R4 and 36 µg compound 2. The ternary complex was prepared in 0.5 mM HEPES at pH 6.35.
T(1)-C2(0.6):
   12 µg of L-RBD formulated with 40 µg T90R4 and 24 µg compound 2. The ternary complex was prepared in 0.5 mM HEPES at pH 6.35.
T(1)-C2(0.3):
   12 µg of L-RBD formulated with 40 µg T90R4 and 12 µg compound 2. The ternary complex was prepared in 0.5 mM HEPES at pH 6.35.
T(1)-C2(0.15):
   12 µg of L-RBD formulated with 40 µg T90R4 and 6 µg compound 2. The ternary complex was prepared in 0.5 mM HEPES at pH 6.35.

Mice receiving PBS intranasally (40 µL/mouse) were studied as negative controls. Each group contained six BALB/c mice (n=6).

Mice received one booster immunizations by the same route of administration as in the priming immunization (intranasally) with formulations identical to the priming formulation on day 14 after priming and on day 28 after priming, respectively. Serum samples were collected on day 28. The RBD-specific IgG ELISA was performed as indicated above.

The results are shown in Fig. 14 for RBD-specific IgG antibodies in serum samples (day 28). "T" refers to the poloxamine 90R4 (T90R4) used in the ternary complex, the value indicated in brackets refers to the final concentration of the respective component in µg/µL.

As can be seen in Fig. 14, intranasal inoculation of ternary complexes containing an identical concentration of L-RBD with varying concentrations of T90R4 and compound 2 resulted in the production of RBD-specific IgG antibodies after administration of most of the tested ternary complex formulations.

No concentration dependence was observed between the administered concentration of T90R4 and/or compound 2 and the induced RBD-specific IgG antibody response.

In a further vaccination experiment, BALB/c mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were administered intranasally (20 µL/mouse) as a bolus through a 20 µL pipette with a tip (priming vaccination, day 0):

### L-RBD(0.3)-T90R4(2.5)-Compound2(0.3):

6 µg of L-RBD formulated with 50 µg T90R4 and 6 µg compound 2. The ternary complex was prepared in 0.5 mM HEPES at pH 6.35.

### L-RBD(0.3) :

6 µg of L-RBD formulated in 20 µL 0.5 mM HEPES solution (pH 6.35).

### PBS:

20 µL PBS per mouse.

Mice received two booster immunizations by the same route of administration as in the priming immunization (intranasally) with formulations identical to the priming formulation on day 14 and day 28 after priming. Mice were sacrificed on day 35 for assessing respiratory cellular immune response. Mouse lungs were harvested, being processed into scraps and digested with collagenase II (0.5 mg/mL) in calcium chloride (1 mM) and magnesium chloride (1 mM) solution at 37 °C for 1 h under shaking at 220 rpm. Next, the samples were filtered through a 75 mm cell strainer to obtain a single cell suspension. The immune cells were obtained by density gradient centrifugation with Percoll (17-0891-09, GE Healthcare, USA) according to the manufacture's instruction.

An Enzyme linked immunospot (ELISpot) assay was performed as follows: Cellular immune response analysis was performed using mouse IFN-γ/IL-4/IL-17 ELISPOT PLUS plates (3321-4AST-2/3311-4APW-2, MABTECH, Sweden). 96-well ELISPOT plates were pre-treated according to the manufacturer's instructions. Mouse pulmonary lymphocytes were plated into each well and stimulated with the following 14-mer overlapping peptide pools spanning the SARS-CoV-2 RBD protein at a final concentration of 1 µg of each peptide per well:

**14-mer overlapping peptide pools spanning the SARS-CoV-2 RBD protein**

| NO | Sequence | NO | Sequence |
|---|---|---|---|
| 1 | RVQPTESIVRFPNI | 23 | FTGCVIAWNSNNLD |
| 2 | ESIVRFPNITNLCP | 24 | IAWNSNNLDSKVGG |
| 3 | FPNITNLCPFGEVF | 25 | NNLDSKVGGNYNYL |
| 4 | NLCPFGEVFNATRF | 26 | KVGGNYNYLYRLFR |
| 5 | GEVFNATRFASVYA | 27 | YNYLYRLFRKSNLK |
| 6 | ATRFASVYAWNRKR | 28 | RLFRKSNLKPFERD |
| 7 | SVYAWNRKRISNCV | 29 | SNLKPFERDISTEI |
| 8 | NRKRISNCVADYSV | 30 | FERDISTEIYQAGS |
| 9 | SNCVADYSVLYNSA | 31 | STEIYQAGSTPCNG |
| 10 | DYSVLYNSASFSTF | 32 | QAGSTPCNGVEGFN |
| 11 | YNSASFSTFKCYGV | 33 | PCNGVEGFNCYFPL |
| 12 | FSTFKCYGVSPTKL | 34 | EGFNCYFPLQSYGF |
| 13 | CYGVSPTKLNDLCF | 35 | YFPLQSYGFQPTNG |
| 14 | PTKLNDLCFTNVYA | 36 | SYGFQPTNGVGYQP |
| 15 | DLCFTNVYADSFVI | 37 | PTNGVGYQPYRVVV |
| 16 | NVYADSFVIRGDEV | 38 | GYQPYRVVVLSFEL |
| 17 | SFVIRGDEVRQIAP | 39 | RVVVLSFELLHAPA |
| 18 | GDEVRQIAPGQTGK | 40 | SFELLHAPATVCGP |
| 19 | QIAPGQTGKIADYN | 41 | HAPATVCGPKKSTN |
| 20 | QTGKIADYNYKLPD | 42 | VCGPKKSTNLVKNK |
| 21 | ADYNYKLPDDFTGC | 43 | KSTNLVKNKCVNF |
| 22 | KLPDDFTGCVIAWN | | |

Additionally, wells incubated with PMA/lonomycin served as a positive control and wells incubated with RPMI 1640 media served as a negative control. After incubation at 37 °C, 5 % CO₂ for 24 h, the plates were washed with PBS followed by incubation with a biotinylated anti-mouse IFN-gamma antibody, with a biotinylated anti-mouse IL-4 antibody or with a biotinylated anti-mouse IL-17 antibody for 2 h at room temperature. Finally, 3-amino-9-ethylcarbazole (AEC) substrate solution were added to visualize the spots. Spots were scanned and quantified by an ImmunoSpot CTL (Bio-Rad) reader. Spot-forming unit (SFU) per million cells was calculated by subtracting the negative control wells.

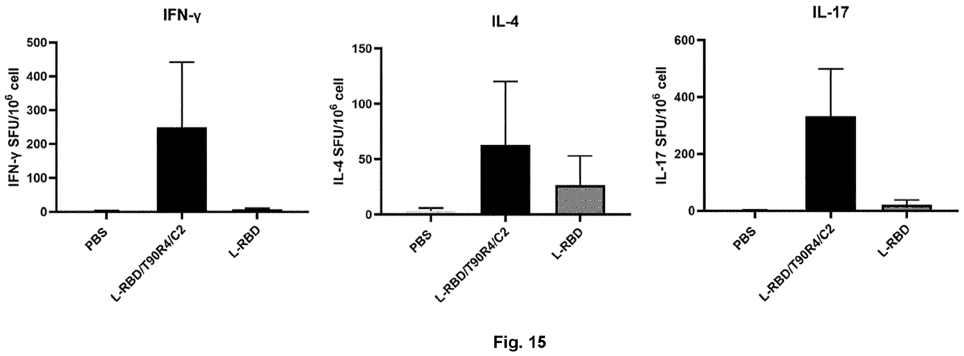

The results are shown in Fig. 15 for RBD-specific IFN-gamma, IL-4, and IL-17 of pulmonary lymphocytes after stimulation with a peptide pool of 14-mer overlapping peptides spanning the SARS-CoV-2 RBD protein.

As can be seen in Fig. 15, intranasal immunization with the ternary complex L-RBD/T90R4/C2 established RBD-specific T-cell responses and cytokine secretion in a pulmonary lymphocyte population of immunized mice. The intranasal immunization with the ternary complex L-RBD/T90R4/C2 resulted in a significantly higher secretion of IFN-gamma and IL-17 compared to the groups receiving intranasal immunizations with L-RBD or PBS. Furthermore, also the secretion of IL-4 was elevated after intranasal immunization with the L-RBD/T90R4/C2 based ternary complex.

In a further vaccination experiment, C57BL/6 mice were anesthetized by intraperitoneal injection with a mixture of medetomidine (0.5 mg/kg), midazolam (5 mg/kg) and fentanyl (0.05 mg/kg) dissolved in PBS. After anesthesia, the following formulations were administered intranasally (40 µL/mouse) by directly pipetting drops into the left and the right nostrils of mice (priming vaccination, day 0):
P.A:
   12 µg of *Pseudomonas aeruginosa* subunit antigen (PcrV) in 0.5 mM HEPES solution (pH 6.35).
P.A-Ternary:
   12 µg of PcrV formulated with 100 µg T90R4 and 12 µg compound 2. The ternary complex was prepared in 0.5 mM HEPES at pH 6.35.

Mice receiving PBS intranasally (40 µL/mouse) were studied as negative controls. Each group contained five BALB/c mice (n=5).

Mice received two booster immunizations by the same route of administration as in the priming immunization with formulations identical to the priming formulation on day 14 after priming and on day 28 after priming, respectively. On days 21, 28 and 42, serum samples and vaginal lavage fluid samples were collected. On day 42, mice were sacrificed and BALF samples and pulmonary tissue samples were collected. PcrV-specific IgG antibodies were determined in serum samples (days 21, 28 and 42). PcrV-specific IgA antibodies were determined in vaginal lavage fluid samples (days 21, 28 and 42) and BALF samples (day 42). The frequencies of CD4+IL-17+ T cells were determined in pulmonary tissue samples (day 42).

Mice were sacrificed on day 42 for assessing the frequencies of pulmonary CD4+ IL-17+ T cells. Mouse lungs were harvested, and digested with DNase I (10 mg/mL, Sigma-Aldrich) and collagenase D (1 mg/mL, Gibco) for 1 h at 37 °C. Then the lungs were passed through a 75 mm cell strainer to obtain a single cell suspension. Subsequently, the erythrocytes were removed by density gradient centrifugation with Percoll, yielding the lung mononuclear cells. The lymphocytes were stimulated with the PcrV protein (0.5 mM) and 5 U/mL IL-2 (PeproTech, Rocky Hill, NJ, USA) in complete RPMI-1640 medium. The medium was removed 2 days later and replaced with fresh medium. Then, the cells were stimulated with leukocyte activation cocktail with GolgiPlug for 4 h. The cell samples were washed and adjusted to a concentration of 5 × 10⁵ cells/mL in flow cytometry buffer (0.5 % FBS in PBS). The samples were first stained for surface markers, followed by intracellular cytokine staining. For surface marker staining, the cells were incubated with the fluorochrome conjugated monoclonal antibodies PerCP/Cyanine5.5 anti-mouse CD4 (GK1.5, BD Biosciences)) and APC/Cyanine7 anti-mouse CD3 (17A2, BD Biosciences). For intracellular cytokine staining, the cells were first incubated with the Cytofix/Cytoperm Fixation/Permeabilization Kit (BD Biosciences) according to the manufacturer's instructions. The samples were then incubated with fluorochrome-conjugated antibodies against APC anti-mouse IL-17A (TC11-18H10.1, BD Biosciences). Finally, the samples were analyzed on a BD FACSArray flow cytometer (BD Biosciences).

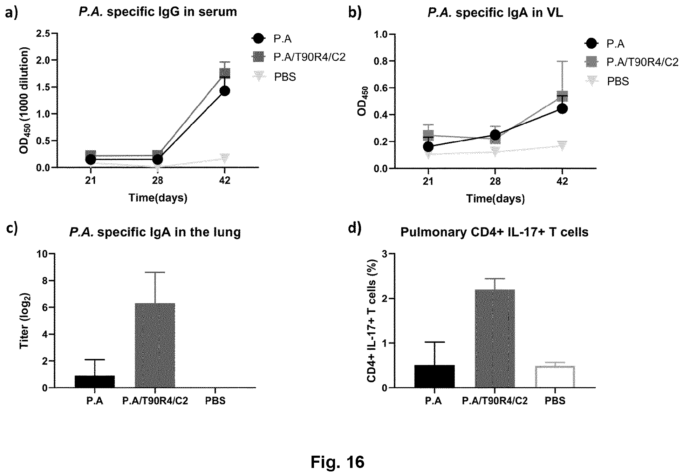

Results are shown in Fig. 16a for PcrV-specific IgG antibodies in serum samples (day 21, day 28 and day 42), in Fig. 16b for PcrV-specific IgA antibodies in vaginal lavage fluid samples (day 21, day 28 and day 42), in Fig. 16c for PcrV-specific IgA antibodies in BALF samples (day 42) and in Fig. 16d for the frequencies of CD4+IL-17+ T cells in pulmonary tissue samples (day 42).

As shown in Fig. 16a, both P.A protein and P.A/T90R4/C2 based ternary complex induced high levels of PcrV-specific IgG antibodies in serum samples on day 42. Furthermore, in the vaginal lavage fluid samples PcrV-specific IgA antibodies are detected in the P.A/T90R4/C2 at the earliest measured time-point on day 21 and reaches the highest level on day 42 compared to all the other groups (Fig. 16b). As can be seen in Fig. 16c, both P.A protein and P.A/T90R4/C2-based ternary complex administration resulted in the production of PcrV-specific IgA antibodies in the lungs of immunized mice. The PcrV-specific IgA antibodies in the P.A/T90R4/C2 group reaches an average endpoint titer of about 1:64, compared to an average endpoint titer of about 1:2 that observed in the P.A group. These results indicate that administration of the ternary complex results in an activated mucosal and humoral immune response. Furthermore, as can be seen in Fig. 16d, P.A/T90R4/C2-based ternary complex administration also successfully mediated cellular immune response in the lungs of immunized mice. The P.A/T90R4/C2-based ternary complex administration resulted in a significant frequency of CD4+ T cells secreting IL-17 (2.236%±0.243) in a pulmonary lymphocyte population compared to the P.A. group and the PBS control group.

## Claims

1. A composition for delivering a protein or a polypeptide to a eukaryotic cell which composition comprises or consists of a ternary complex consisting of the following components:
A synthetic compound, wherein the synthetic compound comprises or consists of a peptide residue and a hydrophobic moiety covalently linked to the peptide residue, wherein the peptide residue is protein binding or polypeptide binding by comprising or consisting of a protein binding or polypeptide binding moiety, wherein the hydrophobic moiety comprises or consists of lipoic acid residue, lipoamide residue, a tetradecyl residue, a cholesteryl residue, or a further peptide residue being hydrophobic by having a sequence consisting of any of SEQ ID NO. 21 to 29 or consisting of a sequence having a sequence similarity of at least 90 % to any of SEQ ID NO. 21 to 29, wherein the sequence similarity is calculated according to the EMBOSS needle algorithm having an open gap penalty of 10.0, an extended gap penalty of 0.5 and using the Blosum 62 matrix,
either an amphiphilic block copolymer or an amphiphilic lipopeptide,
and
either the protein or the polypeptide.

2. Composition according to claim 1, wherein the protein binding or polypeptide binding moiety is a cationic moiety that binds non-specifically to an anionic moiety of the protein or the polypeptide, wherein the protein binding or polypeptide binding moiety has a sequence consisting of any of SEQ ID NO. 1 to 20 or a sequence having a sequence similarity of at least 90 % to any of SEQ ID NO. 1 to 20, wherein the sequence similarity is calculated according to the EMBOSS needle algorithm having an open gap penalty of 10.0, an extended gap penalty of 0.5 and using the Blosum 62 matrix.

3. Composition according to claim 1, wherein the protein binding or polypeptide binding moiety is an anionic moiety that binds non-specifically to a cationic moiety of the protein or the polypeptide, wherein the protein binding or polypeptide binding moiety has a sequence consisting of any of SEQ ID NO. 104 to 136 or a sequence having a sequence similarity of at least 90 % to any of SEQ ID NO. 104 to 136, wherein the sequence similarity is calculated according to the EMBOSS needle algorithm having an open gap penalty of 10.0, an extended gap penalty of 0.5 and using the Blosum 62 matrix.

4. Composition according to claim 1, wherein the protein binding or polypeptide binding moiety is a moiety that binds specifically to a moiety of the protein or the polypeptide, wherein the protein binding or polypeptide binding moiety comprises at least one His tag having a sequence according to SEQ ID NO. 137 and the protein or the polypeptide comprises an antibody or an antigen binding fragment thereof specifically binding to the His tag, or wherein the protein binding or polypeptide binding moiety comprises at least one FLAG tag having a sequence according to SEQ ID NO. 138 and the protein or the polypeptide comprises an antibody or an antigen binding fragment thereof specifically binding to the FLAG tag, or wherein the protein binding or polypeptide binding moiety comprises at least one HA tag having a sequence according to SEQ ID NO. 139 and the protein or the polypeptide comprises an antibody or an antigen binding fragment thereof specifically binding to the HA tag, or wherein the protein binding or polypeptide binding moiety comprises at least one myc tag having a sequence according to SEQ ID NO. 140 and the protein or the polypeptide comprises an antibody or an antigen binding fragment thereof specifically binding to the myc tag, or wherein the protein binding or polypeptide binding moiety comprises at least one V5 tag having a sequence according to SEQ ID NO. 141 and the protein or the polypeptide comprises an antibody or an antigen binding fragment thereof specifically binding to the V5 tag, or wherein the protein binding or polypeptide binding moiety comprises at least one Avi tag having a sequence according to SEQ ID NO. 142 and the protein or the polypeptide comprises an antibody or an antigen binding fragment thereof specifically binding to the Avi tag, or wherein the protein binding or polypeptide binding moiety comprises at least one Strep-tag II having a sequence according to SEQ ID NO. 143 and the protein or the polypeptide comprises biotin, an antibody or an antigen binding fragment thereof specifically binding to the Strep-tag II, or wherein the protein binding or polypeptide binding moiety comprises at least one S tag having a sequence according to SEQ ID NO. 144 and the protein or the polypeptide comprises an antibody or an antigen binding fragment thereof specifically binding to the S tag, or wherein the protein binding or polypeptide binding moiety comprises at least one E tag having a sequence according to SEQ ID NO. 145 and the protein or the polypeptide comprises an antibody or an antigen binding fragment thereof specifically binding to the E tag.

5. Composition according to any of the preceding claims, wherein the peptide residue further comprises at least one targeting moiety, wherein the targeting moiety is any moiety that causes a direction of the synthetic compound to a position or a structure inside or on the surface of the eukaryotic cell, wherein the targeting moiety comprises or consists of a nuclear localization signal sequence, an epithelium cell ligand, in particular a respiratory epithelium cell ligand or a microfold cell ligand, an immune cell ligand, in particular a macrophage cell ligand or a dendritic cell ligand, an endothelial cell ligand, in particular a lung endothelial cell ligand or a liver endothelial cell ligand, or a skin cell ligand, in particular a dermal fibroblast cell ligand or a keratinocyte cell ligand.

6. Composition according to claim 5, wherein the targeting moiety comprises or consists of mannose or of an oligopeptide or polypeptide residue having a sequence consisting of any of SEQ ID NO. 30 to 51 or a sequence having a sequence similarity of at least 90 % to any of SEQ ID NO. 30 to 51, wherein the sequence similarity is calculated according to the EMBOSS needle algorithm having an open gap penalty of 10.0, an extended gap penalty of 0.5 and using the Blosum 62 matrix.

7. Composition according to any of the preceding claims, wherein the hydrophobic moiety is covalently linked to the amino terminus of the peptide residue or to the carboxy terminus of the peptide residue or to any side chain of any amino acid residue of the peptide residue, in particular the side chain of lysine, leucine, arginine, histidine, glutamic acid or aspartic acid.

8. Composition according to any of the preceding claims, wherein the protein or the polypeptide has a molecular weight in a range of 2 kDa to 300 kDa, in particular in a range of 35 kDa to 140 kDa.

9. Composition according to any of the preceding claims, wherein the amphiphilic block copolymer is a poloxamer or a poloxamine, in particular an ethylene oxide - propylene oxide copolymer with tetraether with (1,2-ethanedinitrilo)tetrakis(propanol), wherein the amphiphilic lipopeptide is Pam₃-CSKKKK, PHC-SKKKK, Pam₂Cys-SKKKK, PamDhc-SKKKK or FSL-1.

10. Composition according to any of the preceding claims, wherein the composition further comprises an immunologic adjuvant, wherein the immunologic adjuvant is selected from a group comprising or consisting of CpG oligodeoxynucleotides, polyinosinic:polycytidylic acid, *Quillaja saponaria* extract (QS-21 extract), monophosphoryl-lipid A, IL-2, IL-12, granulocyte-macrophage colony-stimulating factor (GM-CSF), thymic stromal lymphopoietin (TSLP), cyclic di-adenosine monophosphate (c-di-AMP), cyclic guanosine monophosphate-adenosine monophosphate (cGAMP), an aluminum salt, a zinc salt, a manganese salt, squalene, lipopolysaccharide (LPS), alpha-tocopherol, a polysorbate, a sorbitan ester, a lipid positively charged at pH 7.4, an ionizable lipid, a zwitterionic lipid or flagellin.

11. Composition according to any of the preceding claims, wherein the composition further comprises a pharmaceutically acceptable carrier.

12. Composition according to any of claims 1 to 11 for use as a medicament.

13. Use of the composition according to any of claims 1 to 11 for *in vitro* delivery of the protein or the polypeptide to a eukaryotic cell in cell culture.

14. A method for an *in vitro* delivery of a protein or a polypeptide to a eukaryotic cell in cell culture comprising the following steps:
a1) providing the synthetic compound as specified in any of claims 1 to 7, providing the protein or the polypeptide as specified in any of claims 1 and 8, providing the amphiphilic block copolymer or the amphiphilic lipopeptide as specified in any of claims 1 and 9 and providing an aqueous buffer solution,
b1.1) mixing the synthetic compound with the amphiphilic block copolymer or the amphiphilic lipopeptide in the aqueous buffer solution to allow the formation of a binary complex,
b1.2) mixing the binary complex with the protein or the polypeptide in the aqueous buffer solution to allow the formation of a ternary complex and
c1) contacting the ternary complex with the eukaryotic cell
or
a2) providing the synthetic compound as specified in any of claims 1 to 7, providing the protein or the polypeptide as specified in any of claims 1 and 8, providing the amphiphilic block copolymer or the amphiphilic lipopeptide as specified in any of claims 1 and 9 and providing an aqueous buffer solution,
b2.1) mixing the protein or the polypeptide with the amphiphilic block copolymer or the amphiphilic lipopeptide in the aqueous buffer solution to allow the formation of a binary complex,
b2.2) mixing the binary complex with the synthetic compound in the aqueous buffer solution to allow the formation of a ternary complex and
c2) contacting the ternary complex with the eukaryotic cell.
